Europäisches Patentamt
European Patent Office
Office européen des brevets

(19)

(11) Publication number: **0 160 969 A2**

(12) # EUROPEAN PATENT APPLICATION

(21) Application number: 85105579.8

(22) Date of filing: 07.05.85

(51) Int. Cl.⁴: C 07 D 501/46

(30) Priority: 09.05.84 PC T/JP84/00234

(43) Date of publication of application: 13.11.85 Bulletin 85/46

(84) Designated Contracting States: AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Miyake, Akio
30-22, Ominemotomachi 2-chome
Hirakata-shi Osaka 573(JP)

(72) Inventor: Kondo, Masahiro
19-4, Yamasaka 4-chome Higashi-sumiyoshi-ku
Osaka-shi Osaka 546(JP)

(72) Inventor: Fujino, Masahiko
10-7, Hibarigaoka 2-chome
Takarazuka-shi Hyogo 665(JP)

(74) Representative: von Kreisler, Alek, Dipl.-Chem. et al,
Deichmannhaus am Hauptbahnhof
D-5000 Köln 1(DE)

(54) **Cephem compounds.**

(57) A cephem compound of the formula:

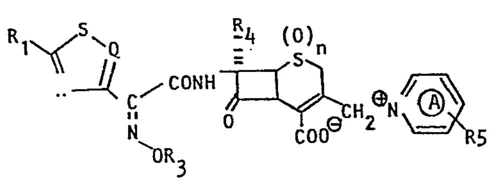

wherein $R_1$ is an optionally protected amino group; Q is a nitrogen atom or a $C{-}R_2$ group; $R_2$ is a hydrogen or halogen atom; $R_3$ is a hydrogen atom or a hydrocarbon residue which may be substituted; $R_4$ is a hydrogen atom or a methoxy group; $R_5$ is a nitrogen-containing aromatic heterocyclic group which may be substituted; A denotes that further substituted or substituents may be present on the pyridine ring; n is 0 or 1 or a physiologically or pharmaceutically acceptable salt or ester thereof.

This compound is novel and has an excellent antibacterial activity.

EP 0 160 969 A2

CEPHEM COMPOUNDS

This invention relates to novel cephem compounds which exhibit excellent antimicrobial activities, to a process for producing the same and to pharmaceutical compositions.

This invention relates to a cephem compound of the formula:

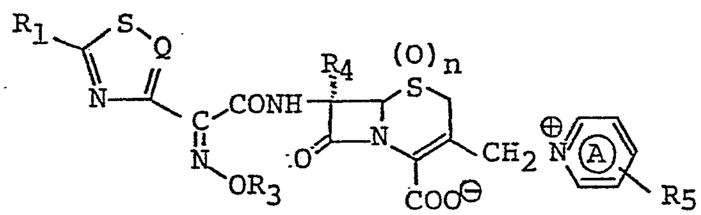

[I]

wherein $R_1$ is an optionally protected amino group; Q is a nitorgen atom or a $C-R_2$ group; $R_2$ is a hydrogen or halogen atom; $R_3$ is a hydrogen atom or an optionally substituted hydrocarbon residue; $R_4$ is a hydrogen atom or a methoxy group; $R_5$ is an optionally substituted nitrogen-containing aromatic heterocyclic group; Ⓐ denotes that further substituent or substituents may be present on the pyridine ring; n is 0 or 1 or its physiologically or pharmaceutically acceptable salt or ester, to a process for producing the same and to pharmaceutical compositions.

The cephem compound as used herein is named on the basis of "cephem" as defined in "The Journal of the American Chemical Society", <u>84</u>, 3400 (1962), and means, among the cephem compounds, one having a double bond in the 3,4-position.

The compounds of this invention have a characteristic feature in having at the 3-position a pyridinium-1-yl group substituted with a nitrogen-containing aromatic heterocyclic group. The present inventors succeeded in synthesizing compounds of the general formula [I] having the characteristics of such a chemical structure, and after investigation of the antimicrobial activity and antimicrobial spectrum of the compounds, found that the compounds [I] process powerful antimicrobial activity against various bacteria, particularly have strong antimicrobial activity against the cephalosporin resistant bacteria as described above and exhibit characteristic antimicrobial action against organisms of the genus Pseudomonas. The findings led to completion of the present invention.

In the above-described cephem compound [I], the substituent $R_1$ is an optionally protected amino group, namely an amino or protected amino group. In the fields of β-lactams and peptide synthesis, the amino protecting groups have been fully studied, and the method of their protection has been already established. In the present invention, as the amino protecting group, there can be suitably used these known ones, and such amino protecting groups include, for example, $C_{6-10}$ aryl-acyl, $C_{1-5}$ alkanoyl, $C_{3-5}$ alkenoyl, $C_{6-10}$ arylsulfonyl, $C_{1-10}$ alkylsulfonyl, substituted oxycarbonyl, carbamoyl, thiocarbamoyl, $C_{6-10}$ aryl-methyl, $C_{6-10}$ aryl-methylene, $C_{6-10}$ arylthio, substituted silyl and 2-$C_{1-8}$ alkoxy-carbonyl-1-methyl-1-ethenyl groups. The $C_{6-10}$ aryl-acyl groups include specifically benzoyl, naphthoyl, phthaloyl, etc., whereby these aromatic rings may be substituted by $C_{1-4}$ alkyl, $C_{1-6}$ alkoxy, halogens, nitro, etc. The substituted aryl-acyl groups includes specifically p-toluoyl, p-tert-butylbenzoyl, p-methoxybenzoyl, p-tert-butoxybenzoyl, p-chlorobenzoyl, p-nitrobenzoyl, etc. The $C_{1-5}$ alkanoyl groups include specifically formyl, acetyl, propionyl, butyryl, valeryl, pivaloyl, succinyl, glutaryl, etc., whereby these alkanoyl groups may be further substituted by $C_{1-6}$ alkoxy, halogens, $C_{6-10}$ aryl, $C_{6-10}$ aryloxy, $C_{6-10}$ arylthio, etc. The substituted alkanoyl groups include specifically mono-

chloroacetyl, dichloroacetyl, trichloroacetyl, monobromoacetyl, monofluoroacetyl, difluoroacetyl, trifluoroacetyl, monoiodoacetyl, acetoacetyl, 3-oxobutyryl, 4-chloro-3-oxobutyryl, phenylacetyl, p-chlorophenylacetyl, phenoxyacetyl, p-chlorophenoxyacetyl, etc. The $C_{3-5}$ alkenoyl groups include specifically acryloyl, crotonoyl, maleoyl, etc., and these alkenoyl groups may be further substituted by $C_{6-10}$ aryl, etc. The substituted alkenoyl groups include specifically cinnamoyl, etc. The $C_{6-10}$ arylsulfonyl groups include specifically benzenesulfonyl, naphthalenesulfonyl, etc, and these aromatic rings may be substituted by $C_{1-4}$ alkyl, $C_{1-6}$ alkoxy, halogens, nitro, etc. The substituted arylsulfonyl groups include specifically p-toluenesulfonyl, p-tert-butylbenzenesulfonyl, p-methoxybenzenesulfonyl, p-chlorobenzenesulfonyl, p-nitrobenzenesulfonyl, etc. The $C_{1-10}$ alkylsulfonyl groups include specifically methanesulfonyl, ethanesulfonyl, camphorsulfonyl, etc., and these alkylsulfonyl groups may be substituted by $C_{6-10}$ aryl, $C_{6-10}$ aryloxy, halogens, etc. The substituted oxycarbonyl groups include $C_{1-8}$ alkoxy-carbonyl groups, such as methoxycarbonyl, ethoxycarbonyl, isopropoxycarbonyl, tert-butoxycarbonyl and isobornyloxycarbonyl, $C_{6-10}$ aryloxycarbonyl groups, such as phenoxycarbonyl and naphthyloxycarbonyl, and $C_{7-12}$ aralkyloxy-carbonyl groups, such as benzyloxycarbonyl. The $C_{1-8}$ alkoxy-carbonyl groups may be further substituted by $C_{1-6}$ alkoxy, $C_{2-5}$ alkanoyl, substituted silyl, $C_{1-4}$ alkylsulfonyl, halogens, cyano, etc. The substituted alkoxycarbonyl groups include, for example, methoxymethyloxycarbonyl, acetylmethyloxycarbonyl, 2-trimethylsilylethoxycarbonyl, 2-methanesulfonylethoxycarbonyl, 2,2,2-trichloroethoxycarbonyl, 2-cyanoethoxycarbonyl, etc. The aromatic rings of the $C_{6-10}$ aryloxy-carbonyl and $C_{7-10}$ aralkyloxycarbonyl groups may be substituted by $C_{1-4}$ alkyl, $C_{1-6}$ alkoxy, halogens, nitro, etc. The substituted aryloxycarbonyl groups include specifically p-methylphenoxycarbonyl, p-methoxyphenoxycarbonyl, p-chlorophenoxycarbonyl, etc. The substituted aralkyloxycarbonyl groups include specifically

p-methylbenzyloxycarbonyl, p-methoxybenzyloxycarbonyl, p-chlorobenzyloxycarbonyl, p-nitrobenzyloxycarbonyl, etc. The alkyl groups of the $C_{7-12}$ aralkyloxy-carbonyl groups may be substituted by $C_{6-10}$ aryl, halogens, etc., and such aralkyloxycarbonyl groups include specifically benzhydryloxycarbonyl, etc. The carbamoyl groups may be substituted. The substituted carbamoyl groups include specifically N-methylcarbamoyl, N-ethylcarbamoyl, N,N-dimethylcarbamoyl, N-phenylcarbamoyl, N-(p-methoxyphenyl)carbamoyl, etc. The thiocarbamoyl groups may be substituted likewise. The substituted thiocarbamoyl groups include specifically N-methylthiocarbamoyl, etc. The $C_{6-10}$ aryl-methyl groups include specifically benzyl, naphthylmethyl, etc., whereby these aromatic rings may be substituted by $C_{1-4}$ alkyl, $C_{1-6}$ alkoxy, halogens,nitro, etc. The substituted arylmethyl groups include specifically p-methylbenzyl, p-methoxybenzyl, p-chlorobenzyl, p-nitrobenzyl, etc. The methyl groups of the arylmethyl groups may be substituted by further 1 to 2 $C_{6-10}$ aryl groups, and such substituted arylmethyl groups include specifically benzhydryl, trityl, etc. The $C_{6-10}$ aryl-methylene groups include specifically benzylidene, etc., and these aromatic rings may be substituted by $C_{1-4}$ alkyl, $C_{1-6}$ alkoxy, halogens, nitro, etc. The substituted arylmethylene groups include specifically p-methylbenzylidene, p-chlorobenzylidene, etc. The $C_{6-10}$ arylthio groups include specifically o-nitrophenylthio, etc. The substituted silyl groups denote silyl groups which combine with the amino group to be protected to be represented by the general formula $R_6R_7R_8SiNH$, $(R_6R_7R_8Si)_2N$ or $Z'\langle{}^{Si(R_9R_{10})}_{Si(R_{9'}R_{10'})}\rangle N$ wherein $R_6$, $R_7$, $R_8$, $R_9$, $R_{10}$, $R_{9'}$ and $R_{10'}$ independently represent a $C_{1-4}$ alkyl group, such as methyl, ethyl and tert-butyl, or a $C_{6-10}$ aryl group, such as phenyl, and may be the same or different; Z' is a $C_{1-3}$ alkylene group, such as methylene and ethylene , and include specifically trimethylsilyl, tert-butyldimethylsilyl, $-Si(CH_3)_2CH_2CH_2Si(CH_3)_2-$, etc. The $C_{1-8}$ alkoxy groups of the 2-$C_{1-8}$ alkoxy-carbonyl-methyl-1-ethenyl groups include, for example, methoxy, ethoxy, tert-butoxy, etc.

In the above-described cephem compounds [I], the symbol Q is a nitrogen atom or a C-$R_2$ group wherein the substituent $R_2$ is a hydrogen or halogen atom. The halogen atom includes fluorine, chlorine, bromine, etc., preferably chlorine.

In the above-described cephem compounds [I], the substituent $R_3$ is a hydrogen atom or an optionally substituted hydrocarbon residue. The hydrocarbon residue includes, for example, $C_{1-6}$ alkyl, $C_{2-6}$ alkenyl and $C_{3-6}$ cycloalkyl group, especially preferably, $C_{1-3}$ alkyl group(methyl, ethyl, propyl) or a substituted $C_{1-3}$ alkyl group. The $C_{1-6}$alkyl groups include specifically methyl, ethyl, propyl, isopropyl, butyl, isobutyl, sec-butyl, tert-butyl, pentyl, hexyl, etc. The $C_{2-6}$ alkenyl groups include specifically vinyl, allyl, isopropenyl, methallyl, 1,1-dimethylallyl, 2-butenyl, 3-butenyl, etc. The $C_{3-6}$ cycloalkyl groups include specifically cyclopropyl, cyclobutyl, cyclopentyl, cyclohexyl, etc. The substituents in these hydrocarbon residues include, for example, hydroxyl group, $C_{3-6}$ cycloalkyl groups, $C_{1-6}$ alkoxy groups, $C_{3-6}$ cycloalkyloxy groups, $C_{6-10}$ aryloxy groups, $C_{7-12}$ aralkylxoy groups, mercapto group , $C_{1-6}$ alkylthio groups, $C_{3-6}$ cycloalkylthio groups, $C_{6-10}$ arylthio groups, $C_{7-12}$ aralkylthio, amino group , mono-$C_{1-4}$ alkylamino groups, di-$C_{1-4}$ alkylamino groups, $C_{3-6}$ cycloalkylamino groups, $C_{6-10}$ arylamino groups, $C_{7-12}$ aralkylamino groups, cyclic amino groups, azide group , nitro group, halogen atoms, cyano group , carboxyl group, $C_{1-8}$ alkoxy-carbonyl groups, $C_{6-10}$ aryloxy-carbonyl groups, $C_{3-6}$ cycloalkyloxy-carbonyl groups, $C_{7-12}$ aralkyloxy-carbonyl groups, $C_{6-10}$ aryl-acyl groups, $C_{1-5}$ alkanoyl groups, $C_{3-5}$ alkenoyl groups, $C_{6-10}$ aryl-acyloxy groups, $C_{2-5}$ alkanoyloxy groups, $C_{3-5}$ alkenoyloxy groups, carbamoyl group , substituted carbamoyl groups, thiocarbamoyl group , substituted thiocarbamoyl groups, carbamoyloxy group , substituted carbamoyloxy groups, phthalimido group , $C_{2-5}$ alkanoylamido groups, $C_{6-10}$ aryl-acylamido groups, carboxyamino group , $C_{1-4}$ alkoxy-carbonylamino groups, $C_{6-10}$ aryloxy-carbonylamino groups, $C_{7-12}$ aralkyloxy-carbonyl-

amino groups, etc. Specifically, the $C_{3-6}$ cycloalkyl groups comprehend cyclopropyl, cyclobutyl, cyclopentyl, cyclohexyl, etc.; the $C_{1-6}$ alkoxy groups comprehend methoxy, ethoxy, propoxy, isopropoxy, butoxy, tert-butoxy, etc.; the $C_{3-6}$ cycloalkyloxy groups comprehend cyclopropyloxy, cyclohexyloxy, etc.; the $C_{6-10}$ aryloxy groups comprehend phenoxy, naphthyloxy, etc.; the $C_{7-12}$ aralkyloxy groups comprehend benzyloxy, 2-phenylethyloxy, etc.; the $C_{1-6}$ alkylthio groups comprehend methylthio, ethylthio, propylthio, butylthio, etc.; the $C_{3-6}$ cycloalkylthio groups comprehend cyclopropylthio, cyclohexylthio, etc.; the $C_{6-10}$ arylthio groups comprehend phenylthio, etc.; the $C_{7-12}$ aralkylthio groups comprehend benzylthio, etc; the mono-$C_{1-4}$ alkylamino groups comprehend methylamino, ethylamino, propylamino, butylamino, etc.; the di-$C_{1-4}$ alkylamino groups comprehend dimethylamino, diethylamino, methyl--ethylamino, dipropylamino, dibutylamino, etc.; the $C_{3-6}$ cycloalkylamino groups comprehend cyclopropylamino, cyclohexylamino, etc..; the $C_{6-10}$ arylamino groups comprehend anilino, etc.; the $C_{7-12}$ aralkylamino groups comprehend benzylamino, 2-phenylethylamino, etc.; the cyclic amino groups comprehend pyrrolidino, piperidino, piperazino, mopholino, 1-pyrrolyl, etc.; the halogen atoms comprehend fluorine, chlorine, bromine, etc.; the $C_{1-8}$ alkoxy-carbonyl groups comprehend methoxycarbonyl, ethoxycarbonyl, propoxycarbonyl, isopropoxycarbonyl, butoxycarbonyl, tert-butoxycarbonyl, isobornyloxycarbonyl, etc.; the $C_{6-10}$ aryloxy-carbonyl groups comprehend phenoxycarbonyl, etc.; the $C_{3-6}$ cycloalkyloxy-carbonyl groups comprehend cyclopropyloxycarbonyl, cyclohexyloxycarbonyl, etc.; the $C_{7-12}$ aralkyloxy-carbonyl group comprehend benzyloxycarbonyl, etc.; the $C_{6-10}$ aryl-acyl groups comprehend benzoyl, phthaloyl, phenylacetyl, etc.; the $C_{1-5}$ alkanoyl groups comprehend formyl, acetyl, propionyl, butyryl, valeryl, pivaloyl, etc.; the $C_{3-5}$ alkenoyl groups comprehend acryloyl, crotonoyl, maleoyl, etc.; the $C_{6-10}$ aryl-acyloxy groups comprehend benzoyloxy, etc.; the $C_{2-5}$ alkanoyloxy groups comprehend acetoxy, propionyloxy, butyryloxy, pivaloyloxy, etc.; the $C_{3-5}$ alkenoyloxy groups comprehend acryloyloxy, etc.; the substituted carbamoyl groups N-methylcarbamoyl, N,N-dimethylcarbamoyl, N-ethylcarbamoyl, N-phenylcarbamoyl, pyrrolidinocarbonyl, piperizinocarbonyl, piperazinocarbonyl, morpholinocarbonyl, etc.; the substituted thiocarbamoyl groups comprehend

N-methylthiocarbamoyl, etc.; the substituted carbamoyloxy groups comprehend N-methylcarbamoyloxy, N,N-dimethylcarbamoyloxy, N-ethylcarbamoyloxy, etc.; the $C_{2-5}$ alkanoylamido groups comprehend acetamido, propionamido, etc.; the $C_{6-10}$ aryl-acylamido groups comprehend benzamido, etc.; the $C_{1-4}$ alkoxy-carbonylamino groups comprehend methoxycarbonylamino, ehtoxycarbonylamino, tert-butoxycarbonylamino, etc.; the $C_{6-10}$ aryloxy-carbonylamino groups comprehend phenoxycarbonylamino, etc.; and the $C_{7-12}$ aralkylxoy-carbonylamino groups comprehend benzyloxycarbonylamino, etc., respectively. The alkyl groups which constitute the aralkyl groups in the above described aralkyloxy, aralkylthio, aralkylamino, aralkyloxycarbonyl and aralkyloxycarbonylamino groups may be substituted by another one $C_{6-10}$ aryl group, and there may be specifically mentioned benzhydryloxy, benzhydrylthio, benzhydrylamino, benzhydryloxycarbonyl, benzhydryloxycarbonylamino, etc. As the substituted hydrocarbon residue, more preferable is $C_{1-3}$ alkyl group substituted with halogen atom, hydroxyl group, amino group, $C_{1-6}$ alkoxy group, carboxyl group, $C_{1-6}$ alkoxy-carbonyl group or cyano group. As specific examples of the substituted hydrocarbon residue, there may be mentioned methoxymethyl, 1-methoxyethyl, 1-ethoxyethyl, 2-hydroxyethyl, 2-aminoethyl, 2-fluoroethyl, 2-chloroethyl, 2-bromoethyl, carboxymethyl, 1-carboxy-1-methylethyl, 1-carboxycyclopropyl, methoxycarbonylmethyl, ethoxycarbonylmethyl, 1-methoxycarbonylcyclopropyl, tert-butoxycarbonylmethyl, 1-methoxycarbonyl-1-methylethyl, 1-ethoxycarbonyl-1-methylethyl, 1-tert-butoxycarbonyl-1-methylethyl, 1-benzyloxycarbonyl-1-methylethyl and 1-pivaloyloxycarbonyl-1-methylethyl as well as many other groups. All of the $OR_3$ groups as mentioned herein are in the <u>syn</u> configuration (Z configuration).

In the above-described cephem compounds [I], the substituent $R_4$ is a hydrogen atom or a methoxy group.

In the above-described cephem compounds [I], the substituent $R_5$ is an optionally substituted nitrogen-containing aromatic heterocyclic group. In this case, preferable as the nitrogen-containing aromatic heterocyclic ring are aromatic five-membered heterocyclic rings containing 1 to 4 nitrogen atoms and optionally one oxygen or sulfur atom, whereby such nitrogen-containing aromatic five-membered heterocyclic

rings ~~combine~~ are linked respectively to the pyridine ring through carbon-carbon atoms. Specific examples of such nitrogen-containing aromatic five-membered heterocyclic groups include 2-pyrrolyl, 3-pyrrolyl, 3-pyrazolyl, 4-pyrazolyl, 5-pyrazolyl, 2-imidazolyl, 4-imidazoyl, 5-imidazolyl, 1,2,3-triazol-4-yl, 1,2,3-triazol-5-yl, 1,2,4-triazol-3-yl, 1,2,4-triazol-5-yl, 1H-tetrazolyl, 3-isoxazolyl, 4-isoxazolyl, 5-isoxazolyl, 2-oxazolyl, 4-oxazolyl, 5-oxazolyl, 1,2,3-oxadiazol-4-yl, 1,2,3-oxadiazol-5-yl, 1,2,4-oxadiazol-3-yl, 1,2,4-oxadiazol-5-yl, 3-furazanyl, 1,3,4-oxadiazol-2-yl, 1,2,3,4-oxatriazol-5-yl, 1,2,3,5-oxatriazol-4-yl, 3-isothiazolyl, 4-isothiazolyl, 5-isothiazolyl, 2-thiazolyl, 4-thiazolyl, 5-thiazolyl, 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl, 1,3,4-thiadiazol-2-yl, 1,2,3,4-thiatriazol-5-yl, 1,2,3,5-thiatriazol-4-yl, etc. The above-mentioned nitrogen-containing aromatic five-membered heterocyclic rings may condense with other aromatic or aromatic-heterocyclic ring. The nitrogen-containing aromatic five-membered heterocyclic ring condensed to other aromatic or aromatic-heterocyclic ring includes specifically 2-indolyl, 3-indolyl, 2-isoindolyl, 3-indazolyl, 2-benzimidazolyl, 2-benzoxazolyl, 2-benzothiazolyl, 8-purinyl, 9-purinyl, 1-indolizinyl, 2-indolizinyl, 3-indolizinyl, etc. Also, the hydrogen atom linked to the carbon or nitrogen atom in the nitrogen-containing aromatic five-membered heterocyclic ring including condensed nitrogen-containing aromatic five-membered heterocyclic ring may be substituted by one or not less than two of the same or different substituents. Such substituents include, for example, hydroxyl group, $C_{1-4}$ hydroxyalkyl groups, $C_{1-6}$ alkyl groups, $C_{2-6}$ alkenyl groups, $C_{2-6}$ alkynyl groups, $C_{4-6}$ alkadienyl groups, $C_{3-6}$ cycloalkyl groups, $C_{3-6}$ cycloalkenyl groups, $C_{3-6}$ cycloalkyl-$C_{1-6}$ alkyl groups, $C_{6-10}$ aryl groups, $C_{7-12}$ aralkyl groups, heterocyclic groups, $C_{1-6}$ alkoxy groups, $C_{1-6}$ alkoxy-$C_{1-4}$ alkyl groups, $C_{3-6}$ cycloalkylxoy groups, $C_{6-10}$ aryloxy groups, $C_{7-12}$ aralkyloxy groups, mercapto group, $C_{1-4}$ mercaptoalkyl groups, sulfo

group, $C_{1-4}$ sulfoalkyl groups, $C_{1-6}$ alkylthio groups, $C_{1-6}$ alkylthio-$C_{1-4}$ alkyl groups, $C_{3-6}$ cycloalkylthio groups, $C_{6-10}$ arylthio groups, $C_{7-12}$ aralkylthio groups, amino group, $C_{1-4}$ aminoalkyl groups, mono-$C_{1-4}$ alkylamino groups, di-$C_{1-4}$ alkylamino groups, mono-$C_{1-4}$ alkylamino-$C_{1-4}$ alkyl groups, di-$C_{1-4}$ alkylamino-$C_{1-4}$ alkyl groups, $C_{3-6}$ cycloalkylamino groups, $C_{6-10}$ arylamino groups, $C_{7-12}$ aralkylamino groups, cyclic amino groups, cyclic-amino-$C_{1-4}$ alkyl groups, cyclic-amino-$C_{1-4}$ alkylamino groups, azido group, nitro group, halogen atoms, $C_{1-4}$ halogenoalkyl groups, cyano group, cyano-$C_{1-4}$ alkyl groups, carboxyl group, carboxy-$C_{1-4}$ alkyl groups, $C_{1-8}$ alkoxy-carbonyl groups, $C_{1-8}$ alkoxy-carbonyl-$C_{1-4}$ alkyl groups, $C_{6-10}$ aryloxy-carbonyl groups, $C_{3-6}$ cycloalkyloxy-carbonyl groups, $C_{7-12}$ aralkyloxy-carbonyl groups, $C_{6-10}$ aryl-acyl groups, $C_{1-5}$ alkanoyl groups, $C_{2-5}$ alkanoyl-$C_{1-4}$alkyl groups, $C_{3-5}$ alkenoyl groups, $C_{6-10}$ aryl-acyloxygroups, $C_{2-5}$ alkanoylxoy groups, $C_{2-5}$ alkanoyloxy-$C_{1-4}$ alkyl groups, $C_{3-5}$ alkenoyloxy groups, carbamoyl group, carbamoyl-$C_{1-4}$ alkyl groups, substituted carbamoyl groups, thiocarbamoyl group, substituted thiocarbamoyl groups, carbamoyloxy group, carbamoyloxy-$C_{1-4}$ alkyl groups, substituted carbamoyloxy groups, phthalimido group, $C_{2-5}$ alkanoylamido groups, $C_{6-10}$ aryl-acylamido groups, sulfonamido group, carboxyamino group, $C_{1-4}$ alkoxy-carbonylamino groups, $C_{6-10}$ aryloxy-carbonylamino groups and $C_{7-12}$ aralkyloxy-carbonylamino groups. Specifically, the $C_{1-4}$ hydroxyalkyl groups comprehend hydroxymethyl, 2-hydroxyethyl, etc.; the $C_{1-6}$ alkyl groups comprehend methyl, ethyl, propyl, isopropyl, butyl, isobutyl, sec-butyl, tert-butyl, pentyl, hexyl, etc.; the $C_{2-6}$ alkenyl groups comprehend vinyl, allyl, isopropenyl, methallyl, 1,1-dimethylallyl, 1-butenyl, 2-butenyl, 3-butenyl, etc.; the $C_{2-6}$ alkynyl groups comprehend ethynyl, 1-propynyl, 2-propynyl, propargyl, etc.; the $C_{4-6}$ alkadienyl groups comprehend 1,3-butadienyl, etc.; the $C_{3-6}$ cycloalkyl groups comprehend cyclopropyl, cyclobutyl, cyclopentyl, cyclohexyl, etc.; the $C_{3-6}$ cycloalkenyl groups comprehend 1-cyclopentenyl, 2-cyclopentenyl, 3-cyclopentenyl, 1-cyclohexenyl, 2-cyclohexenyl, 3-cyclohexenyl, 1,4-cyclohexadienyl, etc.; the $C_{3-6}$ cycloalkyl-$C_{1-6}$ alkyl groups comprehend cyclopentylmethyl, cyclohexylmethyl, etc.; the $C_{6-10}$ aryl groups comprehend phenyl, naphthyl, etc.; the $C_{7-12}$ aralkyl groups comprehend benzyl, phenethyl, etc.; the heterocyclic groups 5- to 8-membered rings, or condensed rings thereto, containing one to several hetero atoms, such as nitrogen (which may be oxidized), oxygen and sulfur atoms, which have a linkage on the carbon atom, such as 2-pyridyl, 3-pyridyl, 4-pyridyl, N-oxido-2-pyridyl, N-oxido-3-pyridyl, N-oxido-4-pyridyl, 2-piperidinyl, 3-piperidinyl, 4-piperidinyl, piperazinyl, 2-pyrazinyl, 2-pyrimidinyl, 4-pyrimidinyl, 5-pyrimidinyl, 3-pyridazinyl, 4-pyridazinyl, 2-pyranyl, 3-pyranyl, 4-pyranyl, 2-thiopyranyl, 3-thiopyranyl, 4-thiopyranyl, 3H-indol-2-yl, 3H-indol-3-yl, 1,2,3-thiadiazol-4-yl, 1,2,4-thiadiazol-5-yl, 1,2,4-thiadiazol-3-yl, 1,3,4-thiadiazolyl, 1,2,5-thiadiazolyl, 1,2,3-triazolyl, 1,2,4-triazolyl, 1H-tetrazolyl, benzopyranyl, 2-furyl, 3-furyl, 2-thienyl, 3-thienyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 2-pyrrolyl, 3-pyrrolyl, 2-pyrrolidinyl, 3-pyrrolidinyl, 2-imidazolyl, 4-imidazolyl, 5-imidazolyl, 3-pyrazolyl, 4-pyrazolyl and 5-pyrazolyl; the $C_{1-6}$ alkoxy groups comprehend methoxy, ethoxy, propoxy, isopropoxy, butoxy, tert-butoxy, etc.; the $C_{1-6}$ alkoxy-$C_{1-4}$ alkyl groups comprehend methoxymethyl, ethoxyethyl, 2-methoxyethyl, etc.; the $C_{3-6}$ cycloalkyloxy groups comprehend cyclopropyloxy, cyclohexyloxy, etc.; the $C_{6-10}$ aryloxy groups comprehend phenoxy, naphthyloxy, etc.; the $C_{7-12}$ aralkyloxy groups comprehend benzyloxy, 1-phenylethyloxy, 2-phenylethyloxy, etc.; the $C_{1-4}$ mercaptoalkyl groups comprehend mercaptomethyl, 2-mercaptoethyl, etc.; the $C_{1-4}$ sulfoalkyl groups comprehend sulfomethyl, 2-sulfoethyl, etc.; the $C_{1-6}$ alkylthio groups comprehend methylthio, ethylthio, propylthio, isopropylthio, butylthio, etc.; the $C_{1-6}$ alkylthio-$C_{1-4}$ alkyl groups comprehend methylthiomethyl, 2-methylthioethyl, etc.; the $C_{3-6}$ cycloalkylthio groups comprehend cyclopropylthio, cycylohexylthio, etc.; the $C_{6-10}$ arylthio groups comprehend phenylthio, etc.; the $C_{7-12}$

aralkylthio groups comprehend benzylthio, etc.; the $C_{1-4}$ aminoalkyl groups comprehend aminomethyl, 2-aminoethyl, etc.; the mono-$C_{1-4}$ alkylamino groups comprehend methylamino, ethylamino, propylamino, butylamino, etc.; the di-$C_{1-4}$ alkylamino groups comprehend dimethylamino, diethylamino, methylethylamino, dipropylamino, dibutylamino, etc.; the mono-$C_{1-4}$ alkylamino-$C_{1-4}$ alkyl groups comprehend methylaminomethyl, ethylaminomethyl, 2-(N-methylamino)ethyl, 3-(N-methylamino)propyl, etc.; the di-$C_{1-4}$ alkylamino-$C_{1-4}$ alkyl groups comprehend N,N-dimethylaminomethyl, N,N-diethylaminomethyl, 2-(N,N-dimethylamino)ethyl, 2-(N,N-diethylamino)ethyl, 3-(N,N-dimethylamino)propyl, etc; the $C_{3-6}$ cycloalkylamino groups comprehend cyclopropylamino, cyclohexylamino etc.; the $C_{6-10}$ arylamino groups comprehend anilino, N-methylanilino, etc.; the $C_{7-12}$ aralkylamino groups comprehend benzylamino, 1-phenylethylamino, 2-phenylethylamino, etc.; the cyclic amino groups comprehend pyrrolidino, piperidino, piperazino, morpholino, 1-pyrrolyl, etc.; the cyclic-amino-$C_{1-4}$ alkyl groups comprehend pyrrolidinomethyl, piperidinomethyl, piperazinomethyl, morpholinomethyl, 2-(morpholino)-ethyl, etc.; the cyclic-amino-$C_{1-4}$ alkylamino groups comprehend pyrrolidinomethylamino, piperidinomethylamino, piperazinomethylamino, morpholinomethylamino, etc.; the halogen atoms comprehend fluorine, chlorine, bromine, etc.; the $C_{1-4}$ halogenoalkyl groups comprehend monofluoromethyl, difluoromethyl, trifluoromethyl, 1-fluoroethyl, 2-fluoroethyl, monochloromethyl, dichloromethyl, trichloromethyl, 1-chloroethyl, 2-chloroethyl, bromomethyl, iodomethyl, etc.; the cyano-$C_{1-4}$ alkyl groups comprehend cyanomethyl, 2-cyanoethyl, etc.; the carboxy-$C_{1-4}$ alkyl groups comprehend carboxymethyl, 1-carboxyethyl, 2-carboxyethyl, etc.; the $C_{1-8}$ alkoxy-carbonyl groups comprehend methoxycarbonyl, ethoxycarbonyl, propoxycarbonyl, isopropoxycarbonyl, butoxycarbonyl, tert-butoxycarbonyl, isobornyloxycarbonyl, etc.; the $C_{1-8}$ alkoxycarbonyl-$C_{1-4}$ alkyl groups comprehend methoxycarbonylmethyl, ethoxycarbonylmethyl, tert-butoxycarbonylmethyl, etc.; the $C_{6-10}$ aryloxy-carbonyl groups comprehend phenoxycarbonyl, etc.; the $C_{3-6}$ cycloalkyloxy-carbonyl groups comprehend cyclopropyloxycarbonyl, cyclohexyloxycarbonyl, etc.; the $C_{7-12}$ aralkyloxy-carbonyl groups comprehend benzyloxycarbonyl, etc.; the

$C_{6-10}$ aryl-acyl groups comprehend benzoyl, phthaloyl, phenylacetyl, etc.; the $C_{1-5}$ alkanoyl groups comprehend formyl, acetyl, propionyl, butyryl, valeryl, pivaloyl, etc.; the $C_{2-5}$ alkanoyl-$C_{1-4}$ alkyl groups comprehend acetylmethyl, 1-acetylethyl, 2-acetylethyl, etc.; the $C_{3-5}$ alkenoyl groups comprehend acryloyl, crotonoyl, maleoyl, etc.; the $C_{6-10}$ aryl-acyloxy groups comprehend benzoyloxy, etc.; the $C_{2-5}$ alkanoyloxy groups comprehend acetoxy, propionyloxy, butyryloxy, pivaloyloxy, etc.; the $C_{2-5}$ alkanoyloxy-$C_{1-4}$ alkyl groups comprehend acetoxymethyl, 1-acetoxyethyl, 2-acetoxyethyl, etc.; the $C_{3-5}$ alkenoyloxy groups comprehend acryloyloxy, etc.; the carbamoyl-$C_{1-4}$ alkyl groups comprehend carbamoylmethyl, etc.; the substituted carbamoyl groups comprehend N-methylcarbamoyl, N,N-dimethylcarbamoyl, N-ethylcarbamoyl, N,N-diethylcarbamoyl, N-phenylcarbamoyl, piperidinocarbonyl, piperazinocarbonyl, morpholinocarbonyl, etc.; the carbamoyloxy-$C_{1-4}$ alkyl groups comprehend carbamoyloxymethyl, etc.; the substituted thiocarbamoyl groups comprehend N-methylthiocarbamoyl, etc.; the substituted carbamoyloxy groups comprehend N-methylcarbamoyloxy, N,N-dimethylcarbamoyloxy, N-ethylcarbamoyloxy, etc.; the $C_{2-5}$ alkanoylamido groups comprehend acetamido, propionamido, etc.; the $C_{6-10}$ aryl-acylamido groups comprehend benzamido, etc.; the $C_{1-4}$ alkoxy-carbonylamino groups comprehend methoxycarbonylamino, ethoxycarbonylamino, tert-butoxycarbonylamino, etc.; the $C_{6-10}$ aryloxy-carbonylamino groups comprehend phenoxycarbonylamino, etc.; and, the $C_{7-12}$ aralkyloxy-carbonylamino groups comprehend benzyloxycarbonylamino, etc., respectively.The alkyl groups, which constitute the aralkyl groups in the above-described $C_{7-12}$ aralkyl, $C_{7-12}$ aralkyloxy, $C_{7-12}$ aralkythio, $C_{7-12}$ aralkylamino, $C_{7-12}$ aralkyloxy-carbonyl and $C_{7-12}$ aralkyloxycarbonylamino groups, may be substituted by another $C_{6-10}$ aryl group, and there may be specifically mentioned benzhydryl, benzhydryloxy, benzhydrylthio, benzhydrylamino, benzhydryloxycarbonyl, benzhydryloxycarbonylamino, etc.

In the above-described cephem compounds [I], Ⓐ denotes that further substituents may be present on the pyridine ring. Such substituents may be one or two to four of the same or different substituents, and those exemplified and specifically mentioned as the substituents on the nitrogen-containing

aromatic heterocyclic ring of the above $R_5$ can apply here. In addition, Ⓐ comprehends pyridine rings condensed with other alicyclic, aromatic and heterocyclic rings. Their examples include, for example, the following;

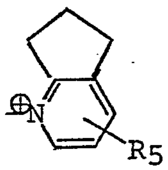 , 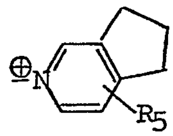 , 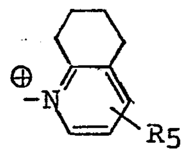 ,

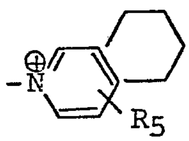 , 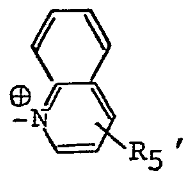 , 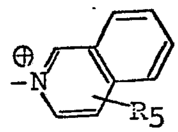 ,

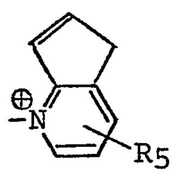 , 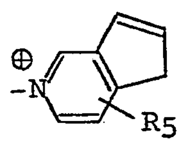 , 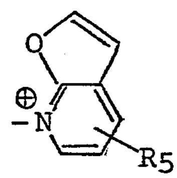 ,

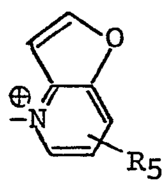 , 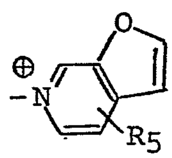 , 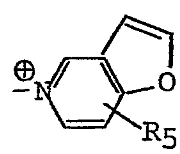 ,

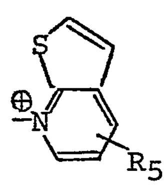 , 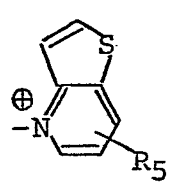 , 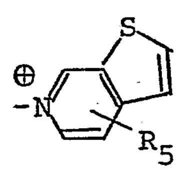 ,

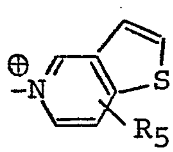 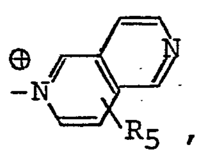 , 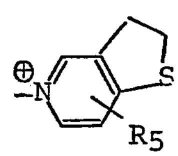

whereby the alicyclic, aromatic and heterocyclic rings condensed with the pyridine ring may be further substituted. As these substituents, those exemplified and specifically mentioned as the substituents on the nitrogen-containing aromatic heterocyclic ring of the above $R_5$ can apply here, and in addition to those, there may be mentioned oxo group, thioxo group, alkylidene groups (e.g., methylene, ethylidene, isopropylidene, etc.) and the like.

In the above-described cephem compounds [I], n is 0 or 1, and the symbol ⊕ put on the nitrogen atom of the pyridine ring and the symbol ⊖ put on the carboxyl group (-COO) at the 4-position mean that the compounds [I] form an inner salt, i.e. pyridinium carboxylate, within the molecule. On the other hand, the compounds [I] may be a physiologically or pharmaceutically acceptable salt or ester. As the physiologically or pharmaceutically acceptable salt, there may be mentioned, for example, inorganic base salts, ammonium salt, organic base salts, inorganic acid addition salts, organic acid addition salts, basic amino-acid salts, etc. The inorganic bases which can form the inorganic base salts include alkali metals (e.g., sodium, potassium, etc.), alkaline earth metals (e.g., calcium, etc.) and the like; the organic bases capable of producing the organic base salts e.g. procaine, 2-phenylethylbenzylamine, dibenzylethylenediamine, ethanolamine, diethanolamine, trishydroxymethylaminomethane, polyhydroxyalkylamine, N-methylglucosamine, etc.; the inorganic acids capable of forming the inorganic acid addition salts e.g. hydrochloric acid, hydrobromic acid, sulfuric acid, nitric acid, phosphoric acid, etc.; the organic acids capable of producing the organic acid addition salts e.g. p-toluenesulfonic acid, methanesulfonic acid, formic acid, trifluoroacetic acid, maleic acid, etc.; and the basic amino acids capable of forming the basic amino-acid salts e.g. lysine, arginine, ornithine, histidine, etc.,

Among these salts, basic salts (i.e. inorganic base salts, ammonium salt, organic base salts and basic amino acid salts) mean salts derivable in case there is acidic group e.g. carboxyl group, sulfo group etc. on the substituent $R_3$ or $R_5$ of the compound [I], and acid addition salts (i.e. inorganic acid addition salts and organic acid addition salts) mean salts derivable in case there is basic group e.g. amino group, monoalkylamino group, dialkylamino group, cycloalkylamino group, arylamino group, aralkylamino group, cyclic amino group, arylamino group, aralkylamino group, cyclic amino group, nitrogen-containing heterocyclic group etc. on the substituent $R_3$ or $R_5$ of the compound [I].

The acid addition salt also includes another type of inner salt of the compound [I], i.e. a salt having carboxyl(COOH) group at the 4-position and 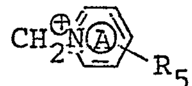 $\cdot M^{\ominus}$ group wherein $M^{\ominus}$ denotes an anion formed by removal of a proton ($H^{\oplus}$) from inorganic acid or organic acid; such an anion is exemplified by chloride ion, bromide ion, sulfate ion, p-toluenesulfonate ion, methanesulfonate ion, trifluoroacetate ion at the 3-position, which is formed through addition of one mole of the said organic or inorganic acid to carboxylate($COO^{\ominus}$) group of the 4-position and 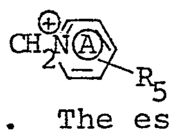 group of the 3-position of the compound [I]. The ester derivatives of the compounds [I] denote esters which can be formed by esterifying the carboxyl group contained in the molecule, and are esters utilizable as a synthetic intermediate and metabolically unstable, nontoxic esters. The esters utilizable as a synthetic intermediate include $C_{1-4}$ alkyl esters, $C_{2-4}$ alkenyl esters, $C_{1-6}$ cycloalkyl esters, $C_{3-6}$ cycloalkyl-$C_{1-4}$ alkyl esters, $C_{6-10}$ aryl esters, $C_{7-12}$ aralkyl esters, substituted silyl esters, etc., whereby these may be further substituted. The alkyls forming the $C_{1-4}$ alkyl esters comprehend specifically methyl, ethyl, propyl, butyl, tert-butyl, etc.; the alkenyls forming the $C_{2-4}$ alkenyl esters comprehend vinyl, allyl, isopropenyl, etc.; the cycloalkyls forming the $C_{3-6}$ cycloalkyl esters comprehend cyclopropyl, cyclobutyl, cyclopentyl, cyclohexyl, etc.; the cycloalkyl-alkyl of the $C_{3-6}$ cycloalkyl-$C_{1-4}$ alkyl esters comprehend cyclopropylmethyl, cyclohexylmethyl, etc.; the aryls forming the $C_{6-10}$ aryl esters comprehend phenyl, etc.; the aralkyls forming the $C_{7-12}$ aralkyl esters comprehend benzyl, phenethyl, etc.; and the substituted silyls forming the substituted silyl esters comprehend trimethylsilyl, tert-butyldimethylsilyl, etc., respectively. The alkyl constituting the aralkyl ester may be substituted by further one to two $C_{6-10}$ aryls, and there may be specifically mentioned benzhydryl ester, trityl ester, etc.

The above esters include ester at the 4-position. Such compound having above ester group at the 4-position forms an intramolecular salt of 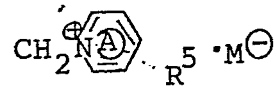 wherein $M^{\ominus}$ has the same meaning as defined above at the 3-position.

The metabolically unstable, nontoxic ester means an ester usable for oral administration and as such ester, there can also be suitably adopted in the present invention those which have already been established in the fields of penicillins and cephalosporins. Such metabolically unstable, nontoxic esters include, for example, $C_{1-5}$ alkanoyloxymethyl esters, $C_{1-5}$ alkanoyloxyethyl esters, $C_{1-6}$ alkoxy-$C_{1-4}$ alkyl esters, $C_{1-6}$ alkylthio-$C_{1-4}$ alkyl esters, etc., and there may be specifically mentioned acetoxymethyl ester, 1-acetoxyethyl ester, 1-acetoxybutyl ester, 2-acetoxyethyl ester, pivaloyloxymethyl ester, methoxymethyl ester, ethoxymethyl ester, isopropoxymethyl ester, 1-methoxyethyl ester, 1-ethoxyethyl ester, methylthiomethyl ester, ethylthiomethyl ester, etc. The present invention embraces the above-described ester derivatives as well as physiologically or pharmaceutically accetable compounds which are converted in vivo into the compounds [I].

The compounds [I] of the present invention possess a broad spectrum of antimicrobial activity, and can be used for the prevention and treatment of various diseases in human beings and animals caused by pathogenic bacteria, such as respiratory tract and urinary tract infections. The compounds [I] provide the following characteristic features of the antimicrobial spectrum:

(1) They exhibit high activity against a great variety of gram-negative bacteria as well as gram-positive bacteria (e.g., Staphylococcus aureus, Corynebacterium diphtheriae, etc.),

(2) They also exhibit high activity against Pseudomonas aeruginosa which does not respond to the treatment with the ordinary cephalosporin antibiotics, and

(3) They have high activity against a number of β-lactamase-producing gram-negative bacteria (e.g. the genera Escherichia, Enterobacter, Serratia, Proteus, etc.). Although the aminoglycoside antibiotics, such as amikacin and gentamicin, have heretofore been employed particularly in diseases caused by microorganisms of the genus Pseudomonas, the compounds [I] not only demonstrate the antimicrobial activity equal to those of the aminoglycoside antibiotics but also are by far lower in toxicities to human and animals than the aminoglycosides, thus providing marked advantages. Besides, the compound [I] has the following characteristic features, i.e. excellent stability, high concentration in blood, long duration of effect and remarkably high concentration in tissue.

The process for producing the cephem compounds [I] of the present invention, their salts or esters is described in detail. The compounds [I] can be produced by the known or *per se* known three methods as mentioned under (1) to (3); namely

(1) By reacting a compound of the formula:

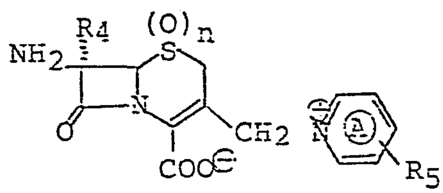

[II]

wherein the symbols are as defined hereinbefore or its salt or ester with a compound of the formula:

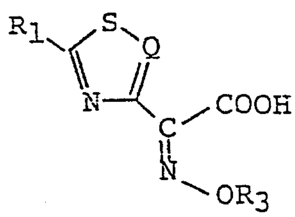

[III]

wherein the symbols are as defined hereinbefore or its reactive derivative, followed by removal of the protective group, if necessary,

(2) By reacting a compound of the formula:

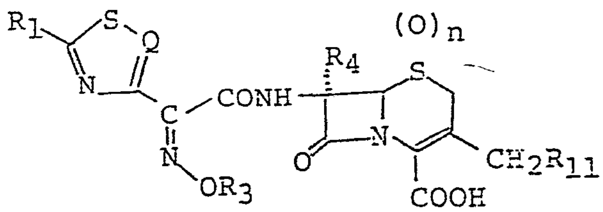

[IV]

wherein $R_{11}$ is a hydroxyl, acyloxy, carbamoyloxy or substituted carbamoyloxy group or a halogen atom, other symbols are as defined hereinbefore or its salt or ester with a pyridine derivative of the formula 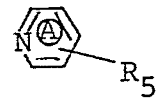

wherein the symbols are as defined hereinbefore or its salt, followed by removal of the protective group, if necessary, or

(3) By reacting a compound of the formula:

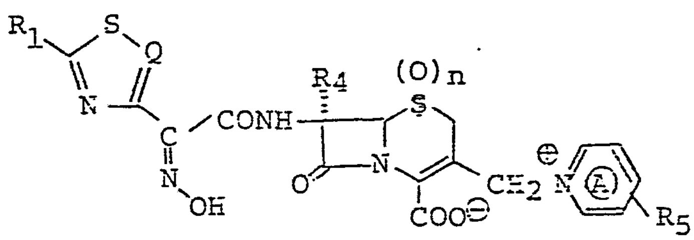

[V]

wherein the symbols are as defined hereinbefore or its salt or ester with a compound of the formula $R_3$'OH wherein $R_{3'}$ is a hydrocarbon residue which may be substituted or its reactive derivative, followed by removal of the protective group, if necessary.

Among the compounds [I], those in which Q is C-$R_2$ can be produced also by the known fourth method as described below under (4).

(4) By reacting a compound of the formula:

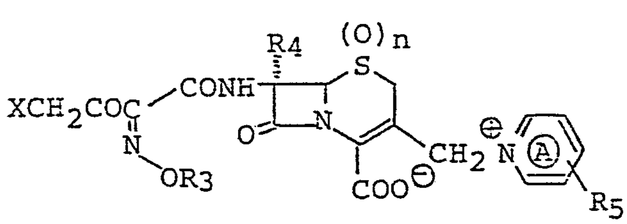

[VI]

wherein X is a halogen atom or a group of the formula $R_6SO_2O$; $R_6$ is a $C_{1-4}$ alkyl group, phenyl group or phenyl substituted with a $C_{1-4}$ alkyl group, $C_{1-6}$ alkoxy group, halogen atom or nitro group; other symbols are as defined hereinbefore or its salt or ester with a compound of the formula $R_1C(=S)NH_2$ wherein $R_1$ is as defined hereinbefore, followed by removal of the protective group, if necessary to give the compound [I] ($Q=C-R_2$). Explanations will be given successively for the production methods (1) to (4), procedure of removal of the protective group and method of purification of the compounds [I].

Production method (1):

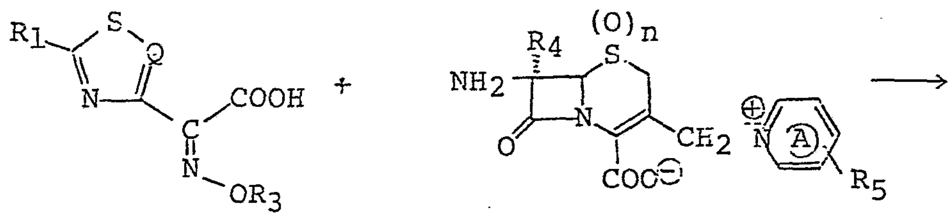

+ → [I]

[III] [II]

This method involves acylation of the 7-amino compound [II] with the carboxylic acid [III] or its reactive derivative. In this method, the carboxylic acid [III] in the free form or its salt or reactive derivative is used as an acylating agent for the 7-amino group in the 7-amino compound [II]. Thus, the free acid [III] or a reactive derivative of the free acid, such as an inorganic salt, organic salt, acid halide, acid azide, acid anhydride, mixed acid anhydride, active amide, active ester and active thioester, is subjected to the acylation reaction. The inorganic salt includes, for example, alkali metal salts (e.g., sodium salt, potassium salt, etc.), alkaline earth metal salts (e.g., calcium salt, etc.), etc.; the organic salt includes trimethylamine salt, triethylamine salt, tert-butyldimethylamine salt, dibenzylmethylamine salt, benzyldimethylamine salt, N,N-dimethylaniline salt, pyridine salt, quinoline salt, etc.; the acid halide includes acid chloride, acid bromide, etc.; the mixed acid anhydride includes mono-$C_{1-4}$ alkylcarbonic acid mixed acid anhydrides (e.g., mixed acid anhydrides of the free acid [III] with monomethylcarbonic acid, monoethylcarbonic acid, monoisopropylcarbonic acid, monoisobutylcarbonic acid, mono-tert-butylcarbonic acid, monobenzylcarbonic acid, mono(p-nitrobenzyl)carbonic acid, monoallylcarbonic acid, etc.), $C_{1-6}$ aliphatic carboxylic acid mixed acid anhydrides (e.g., mixed acid anhydrides of the free acid [III] with acetic acid, cyanoacetic acid, propionic acid, butyric acid, isobutyric acid, valeric acid, isovaleric acid, pivalic acid, trifluoroacetic acid, trichloroacetic acid, acetoacetic acid, etc.), $C_{7-11}$ aromatic carboxylic acid mixed acid anhydrides (e.g., mixed acid anhydrides of the free acid [III] with benzoic acid,

p-toluic acid, p-chlorobenzoic acid, etc.), organic sulfonic acid mixed acid anhydrides (e.g., mixed acid anhydrides with methanesulfonic acid, ethanesulfonic acid, benzenesulfonic acid, p-toluenesulfonic acid, etc.), etc.; and the active amide includes amides with nitrogen-containing heterocyclic compounds (e.g., acid amides of the free acid [III] with pyrazol, imidazole, benzotriazole, etc., whereby these nitrogen-containing heterocyclic compounds may be substituted by $C_{1-4}$ alkyl, $C_{1-6}$ alkoxy, halogen atoms, oxo, thioxo, $C_{1-6}$ alkoxy, etc.), etc., respectively. As the active ester, there can be utilized all of those being usable for such a purpose in the fields of β-lactam and pepetide synthesis, and there may be mentioned, for example, organic phosphates (e.g., diethoxy phosphate, diphenoxy phosphate, etc.) as well as p-nitrophenyl ester, 2,4-dinitrophenyl ester, cyanomethyl ester, pentachlorophenyl ester, N-hydroxysuccinimido ester, N-hydroxyphthalimido ester, 1-hydroxybenzotriazole ester, 6-chloro-1-hydroxybenzotriazole ester, 1-hydroxy-1H-2-pyridone ester, etc. As the active thioester, there may be mentioned esters with aromatic heterocyclic thiol compounds (e.g., 2-pyridylthiol ester, 2-benzothiazolylthiol ester, etc., whereby these heterocyclic rings may be substituted by $C_{1-4}$ alkyl, $C_{1-6}$ alkoxy, halogen atoms, $C_{1-6}$ alkylthio, etc.). On the other hand, the 7-amino compound [II] is used in the free form or as its salt or ester. The salt of the compound [II] includes inorganic base salts, ammonium salts, organic base salts, inorganic acid addition salts, organic acid addition salts, etc. The inorganic base salts include alkali metal salts (e.g., sodium salt, potassium salt, etc.), alkaline earth metal salts (e.g., calcium salt, etc.), etc.; the organic base salts are for example trimethylamine salt, triethylamine salt, tert-butyldimethylamine salt, dibenzylmethylamine salt, benzyldimethylamine salt, N,N-dimethylaniline salt, pyridine salt, quinoline salt, etc.; the inorganic acid addition salts are for example hydrochloride, hydrobromide, sulfate, nitrate, phosphate, etc.; and the organic acid addition salts are for example formate,

acetate, trifluoroacetate, methanesulfonate, p-toluenesulfonate, etc., respectively. As the ester of the compound [II], the esters mentioned hereinbefore as the ester derivative of the compound [I] can apply here. Namely, there may be mentioned $C_{1-4}$ alkyl esters, $C_{2-4}$ alkenyl esters, $C_{3-6}$ cycloalkyl esters, $C_{3-6}$ cycloalkyl-$C_{1-4}$ alkyl esters, $C_{6-10}$ aryl esters, $C_{7-12}$ aralkyl esters, $C_{1-5}$ alkanoyloxymethyl esters, $C_{1-5}$ alkanoyloxyethyl esters and these esters which are further substituted by hydroxyl group, $C_{1-6}$ alkoxy groups, halogen atoms, nitro group, cyano group, mercapto group, $C_{1-6}$ alkylthio group, oxo group, thioxo group, etc. The starting compound [II], its salt and ester as well as the starting compound [III] and its reactive derivative can all be readily produced by the known method or methods similar thereto. The reactive derivative of the compound [III], as a substance isolated from the reaction mixture or as the reaction mixture containing the reaction derivative of the compound [III] without being isolated, can be reacted with the compound [II]. When the carboxylic acid [III] is used in the form of free acid or salt, a suitable condensing agent is employed. As the condensing agent, there are used N,N'-di-substituted carbodiimides, such as N,N'-dicyclohexylcarbodiimide, azolides, such as N,N'-carbonyldiimidazole and N,N'-thiocarbonyldiimidazole, dehydrating agents, such as N-ethoxycarbonyl-2-ethoxy-1,2-dihydroquinoline, phosphorus oxychloride, and alkoxyacetylene, and 2-halogenopyridinium salts, such as 2-chloropyridinium methyliodide and 2-fluoropyridinium methyliodide. When these condensing agents are used, the reaction is assumed to proceed via the reactive derivative of the carboxylic acid [III]. The reaction is normally carried out in a solvent, whereby the solvent which does not affect adversely the reaction is suitably selected. As such a solvent, there are used ethers, such as dioxane, tetrahydrofuran , diethyl ether, tert-butyl methyl ether, diisopropyl ether and ethylene glycol dimethyl ether, esters, such as ethyl formate, ethyl acetate and butyl acetate, halogenated hydrocarbons, such as dichloromethane, chloroform, carbon tetrachloride, trichlene and 1,2-dichloroethane, hydrocarbons, such as n-hexane, benzene and toluene, amides, such as formamide, N,N-dimethylformamide and N,N-dimethylacetamide, ketones, such as acetone, methyl ethyl ketone and methyl isobutyl ketone and nitriles, such as acetonitrile and propionitrile as well as dimethylsulfoxide, sulfolane, hexamethylphosphoramide, water, etc., either solely or as a solvent mixture. The amount of the acylating agent [III] to be used is normally 1 to 5 moles per mole of the compound [II], preferably 1 to 2 moles. The reaction is carried out in the temperature range of -80 to 80°C, preferably -40 to 50°C, most preferably -30 to 30°C. The reaction time ranges normally from 1 minute to 72 hours, preferably from 15 minutes to 3 hours, depending upon the type of the compounds [II] and [III], the kind of solvent (also, the mixing ratio in the case of a solvent mixture used), the reaction temperature, etc. In cases in which an acid halide is used as an acylating agent, the reaction can be carried out in the presence of a deacidifying agent for the purpose of eliminating the liberated hydrogen halide from the reaction system. As such a deacidifying agent, there may be mentioned inorganic bases, such as sodium carbonate, potassium carbonate, calcium carbonate and sodium hydrogencarbonate, tertiary amines, such as triethylamine, tripropylamine, tributylamine, cyclohexyldimethylamine, pyridine, lutidine, γ-collidine, N,N-dimethylaniline, N-methylpiperidine, N-methylpyrrolidine and N-methylmorpholine, alkylene oxides, such as propylene oxide and epichlorohydrin, and the like.

Production method (2):

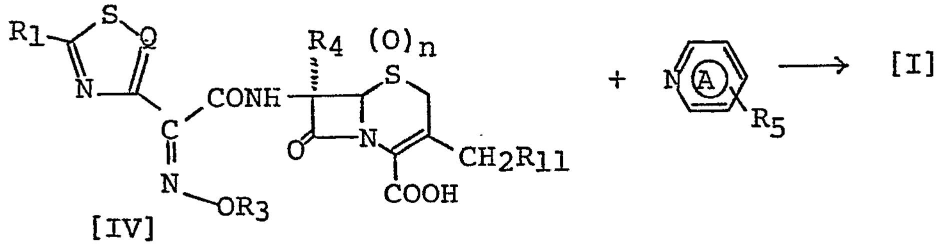

[IV]

This method involves a reaction of the cephem compound

[IV] with a pyridine derivative 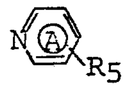 to synthesize the compound [I] through a nucleophilic substitution reaction. In the compound [IV], $R_{11}$ is a hydroxyl, acyloxy, carbamoyloxy or substituted carbamoyloxy group or a halogen atom. With reference to the above, the acyloxy group is $C_{2-5}$ alkanoyloxy or $C_{6-10}$ aryl-acyloxy groups which may be substituted, whereby the $C_{2-5}$ alkanoyloxy groups which may be substituted include specifically acetoxy, chloroacetoxy, propionyloxy, butyryloxy, pivaloyloxy, 3-oxobutyryloxy, 4-chloro-3-oxobutyryloxy, 3-carboxypropionyloxy, 4-carboxybutyryloxy, 3-ethoxycarbamoyl-propionyloxy, etc.; and the $C_{6-10}$ aryl-acyloxy groups which may be substituted include specifically o-carboxybenzoyloxy, o-(ethoxycarbonylcarbamoyl)benzoyloxy, o-(ethoxycarbonylsulfamoyl)benzoyloxy, etc., respectively. The substituted carbamoyloxy group includes specifically methylcarbamoyloxy, N,N-dimethylcarbamoyloxy, etc. The halogen atom includes chlorine, bromine, iodine, etc. The compound [IV] is used in the free form or as its salt or ester. As the salt and ester of the compound [IV], the salts and esters mentioned as the salt and ester of the compound [II] in the production method (1) can apply here. The compound [IV], its salts and esters can be readily produced by the per se known method or methods similar thereto. On the other hand, the nucleophilic reagent 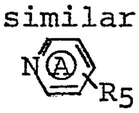 is used as a salt, as well. Such a salt include inorganic acid addition salts, such as hydrochloride, hydrobromide, sulfate, nitrate and phosphate, organic acid addition salts, such as formate, acetate, trifluoroacetate, methanesulfonate and p-toluenesulfonate, and the like. The general synthetic method for the nucleophilic reagent 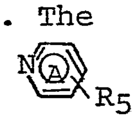 is known, and the production can be easily effected by the method described in the literature or methods similar thereto [for example, V. J. Bauer, H. P. Dalalian, W. J. Fanshawe and S. R. Safir, J. Med. Chem., 11, 981, 984 (1968), etc.]. The present nucleophilic substitution reaction by the above pyridine derivative to the compound [IV] is a reaction well

known per se, and is normally carried out in a solvent. As the solvent to be used in this reaction, the solvents, such as ethers, esters, halogenated hydrocarbons, hydrocarbons, amides, ketones, nitriles and water, which are usable in the production method (1), can apply here, and in addition to these, there are used alcohols, such as methanol, ethanol, propanol, isopropanol, ethylene glycol and 2-methoxyethanol.

(2-1): when $R_{11}$ is an acyloxy, carbamoyloxy or substituted carbamoyloxy group;

More preferable solvent is water or a solvent mixture of water with an organic solvent miscible with water, and among organic solvents miscible with water, more preferable are acetone, methyl ethyl ketone, acetonitrile, etc. The amount of the nucleophilic reagent to be used is normally 1 to 5 moles per mole of the compound [IV], preferably 1 to 3 moles. The reaction is carried out in the temperature range of 10 to 100°C, preferably 30 to 80°C. The reaction time ranges normally from 30 minutes to 5 days, preferably from 1 to 5 hours, depending upon the kinds of the compound [IV] and nucleophilic reagent , the type of the solvent (also, the mixing ratio in the case of a solvent mixture), the reaction temperature, etc. The reaction is advantageously carried out at pH 2 to 8, preferably at a pH in the neighborhood of neutrality or pH 5 to 8. Also, the present reaction more readily proceeds normally in the presence of 2 to 30 equivalents of an iodide or thiocyanate. Such a salt includes sodium iodide, potassium iodide, sodium thiocyanate, potassium thiocyanate, etc. In addition to the above-described salts, quaternary ammonium salts having surface activity, such as trimethylbenzylammonium bromide, triethylbenzylammonium bromide and triethylbenzylammonium hydroxide, in some cases can be added to allow the reaction to proceed smoothly.

(2-2): when $R_{11}$ is a hydroxyl group;

For example, the reaction is carried out in the presence of an organic phosphorus compound in accordance with the method as described in the Japanese Unexamined Patent Publication

No. 4379/1983. The organic phosphorus compound which is useful in the case includes, for example, O-phenylene phosphorochloridate, o-phenylene phosphorofluoridate, methyl o-phenylene phosphate, ethyl o-phenylene phosphate, propyl o-phenylene phosphate, isopropyl o-phenylene phosphate, butyl o-phenylene phosphate, isobutyl o-phenylene phosphate, sec.-butyl o-phenylene phosphate, cyclohexyl o-phenylene phosphate, phenyl o-phenylene phosphate, p-chlorophenyl o-phenylene phosphate, p-acetylphenyl o-phenylene phosphate, 2-chloroethyl o-phenylene phosphate, 2,2,2-trichloroethyl o-phenylene phosphate, ethoxycarbonylmethyl o-phenylene phosphate, carbamoylmethyl o-phenylene phosphate, 2-cyanoethyl o-phenylene phosphate, 2-methylsulfonylethyl o-phenylene phosphate, benzyl o-phenylene phosphate, 1,1-dimethyl-2-propenyl o-phenylene phosphate, 2-propenyl o-phenylene phosphate, 3-methyl-2-butenyl o-phenylene phosphate, 2-thienylmethyl o-phenylene phosphate, 2-furfurylmethyl o-phenylene phosphate, bis-o-phenylene pyrophosphate, 2-phenyl-1,3,2-benzodioxaphosphole-2-oxide, 2-(p-chlorophenyl)-1,3,2-benzodioxaphosphole-2-oxide, 2-butyl-1,3,2-benzodioxaphosphole-2-oxide, 2-anilino-1,3,2-benzodioxaphosphole-2-oxide, 2-phenylthio-1,3,2-benzodioxaphosphole-2-oxide, 2-methoxy-5-methyl-1,3,2-benzodioxaphosphole-2-oxide, 2-chloro-5-ethoxycarbonyl-1,3,2-benzodioxaphosphole-2-oxide, 2-methoxy-5-ethoxycarbonyl-1,3,2-benzodioxaphosphole-2-oxide, 5-ethoxycarbonyl-2-phenyl-1,3,2-benzodioxaphosphole-2-oxide, 2,5-dichloro-1,3,2-benzodioxaphosphole-2-oxide, 4-chloro-2-methoxy-1, 3,2-benzodioxaphosphole-2-oxide, 2-methoxy-4-methyl-1,3,2-benzodioxaphosphole-2-oxide, 2,3-naphthalene methyl phosphate, 5,6-dimethyl-2-methyoxy-1,3,2-benzodioxaphosphole-2-oxide, 2,2-dihydro-4,5,6,7-tetrachloro-2,2,2-trimethoxy-1,3,2-benzodioxaphosphole, 2,2-dihydro-4,5,6,7-tetrachloro-2,2,2-triphenoxy-1,3,2-benzodioxaphosphole, 2,2-dihydro-2,2-ethylenedioxy-2-methoxy-1,3,2-benzodioxaphosphole, 2,2-dihydro-benzyl-2,2-dimethoxy-1,3,2-benzodioxaphosphole, 2,2-dihydro-4,5-benzo-2,2,2-trimethoxy-1,3,2-benzodioxaphosphole, 2,2-dihydro-2,2,2-triphenoxy-1,3,2,-benzodioxaphosphole, 2,2-dihydro-2,2-(o-phenylenedioxy)-2-phenoxy-1,3,2-benzodioxaphosphole, 2-chrolo-2,2,-dihydro-2,2-(o-phenylenedioxy)-1,3,2,-benzodioxaphosphole 2,2-dihydro-2-methoxy-2,2-(o-phenylenedioxy)-1,3,2-benzodioxaphosphole, 2,2-dihydro-2,2,2-trichloro-1,3,2-benzodioxaphosphole, 9,10-phenanthrenedioxytrimethoxyphosphorus, o-phenylen phosphorochloridite, o-phenylene phosphorobromidite, o-phenylene phosphorofluoridite, methyl o-phenylene phoshite, butyl o-phenylene phosphite methoxycarbonylmethyl o-phenylene phosphite, phenyl o-phenylene phosphite,p-chloro (or p-nitro)phenyl o-phenylene phosphite,2-phenyl-1,3,2-benzodioxaphosphole, bis-o-phenylene pyrophosphite, 2-methoxy-5-methyl-1,3,2-benzodioxaphosphole, 5-acetyl-2-phenoxy-1,3,2-benzodioxaphosphole, 9,10-phenanthrene phosphorochloridite, 2-chloro-4-methyl-1,3,2-benzodioxaphosphole,5-ethoxycarbonyl-2-phenyl-1,3,2-benzodioxaphosphole, 2-chloro-2-thioxo-1,3,2-benzodioxaphosphole,2-phenoxy-2-oxo-1,3,2-benzodiazaphosphole,2-phenoxy-1,3,2-benzodioxaaza-phosphole,2,2-dihydro-2-oxo-2-methoxy-4,5-dimethyl-1,3,2-dioxaphosphole,2,2-dihydro-2-oxo-2-chloro-4,5-dimethyl-1,3,2-dioxaphosphole, 2,2-dihydro-2-oxo-2-(1-imidazolyl)-4,5-dimethyl-1,3,2-dioxaphosphole, 2,2-dihydro-2,2-ethylene-dioxy-2-methoxy-4,5-dimethyl-1,3,2-dioxaphosphole, 2,2-dihydro-2,2-dimethoxy-2-phenoxy-4,5-dimethyl-1,3,2-dioxaphosphole, 2,2-dihydro-2,2,2-trimethoxy-4,5-dimethyl-1,3,2-dioxaphosphole, 2,2-dihydro-2,2,2-triphenoxy-4,5-dimethyl-1,3,2-dioxaphosphole, 2,2-dihydro-2,2,2-tirethoxy-4,5-diphenyl-1,3,2-dioxaphosphole, 2,2-dihydro-2,2,2-trimethoxy-4,5-diphenyl-1,3,2-dioxaphosphole, 2,2-dihydro-2-oxo-2-methoxy-4,5-diphenyl-1,3,2-dioxaphosphole, 2,2-dihydro-2,2,2-trimethoxy-1,3,2-dioxaphosphole, 2,2-dihydro-2,2,2-trimethoxy-4-phenyl-1,3,2-dioxaphosphole, 2,2-dihydro-2,2,2,-trimethoxy-4-methyl-1,3,2-dioxaphosphole, 2,2-dihydro-2,2,2-trimethoxy-4-methyl-5-phenylcarbamoyl-1,3,2-dioxaphosphole, 2,2,4,5,6,7 - hexahydro-2,2,2-trimethoxy-1,3,2-benzodioxaphosphole, 2,2'-oxybis(4,5-dimethyl-2,2-dihydro-1,3,2-dioxaphosphole) and 2,2'-oxybis(4,5-dimethyl-2,2-dihydro-1,3,2-dioxaphosphole-2-oxide), etc. The solvent which is useful in the reaction may

be any solvent , unless it affects adversely the reaction, and there may be used preferably the above-described ethers, esters, halogenated hydrocarbons, hydrocarbons, amides, ketones, nitriles, etc., either solely or as a solvent mixture. Particularly, the use of, for example, dichloromethane, acetonitrile, formamide, a solvent mixture of formamide and acetonitrile and a solvent mixture of dichloromethane and acetonitrile can produce satisfactory results. The amounts of the nucleophilic reagent and organic phosphorus compound to be used are 1 to 5 moles and 1 to 10 moles, respectively, per mole of the compound [IV], more preferably 1 to 3 moles and 1 to 6 moles, respectively. The reaction time is normally from 1 minute to 15 hours, preferably from 5 minutes to 2 hours. An organic base may be added to the reaction system. Such an organic base include, for example, amines such as triethylamine, tributylamine, dibutylamine, diisobutylamine, dicyclohexylamine and 2,6-lutidine. The amount of the base to be added desirably is 1 to 5 moles per mole of the compound [IV].

(2-3); when $R_{11}$ is a halogen atom;

The preferable solvent includes the above-described ethers, esters, halogenated hydrocarbons, hydrocarbons, amides, ketones, nitriles, alcohols, water, etc. The amount of the nucleophilic reagent to be used is normally 1 to 5 moles per mole of the compound [IV], preferably 1 to 3 moles. The reaction is carried out in the temperature range of 0 to 80°C, preferably 20 to 60°C. The reaction time ranges normally from 30 minutes to 15 hours, preferably from 1 to 5 hours. In order to promote the reaction, the reaction can also be carried out in the presence of a dehalogenating agent. As such a dehalogenating agent, there may also mentioned here the deacidifying agents as described under the item of the production method (1), such as inorganic bases, tertiary amines and alkylene oxides, but the nucleophilic reagent itself may be allowed to act as a dehalogenating agent. In such a case, the reagent is used in quantities of not less

than 2 moles per mole of the compound [IV]. The halogen atom represented by $R_{11}$ is chlorine, bromine, iodine, etc., and preferably iodine. The compound [IV] wherein $R_{11}$ is iodine can be readily produced, for example, by using the method as described in the Japanese Unexamined Patent Publication No. 57390/1983 or methods similar thereto.

Production method (3):

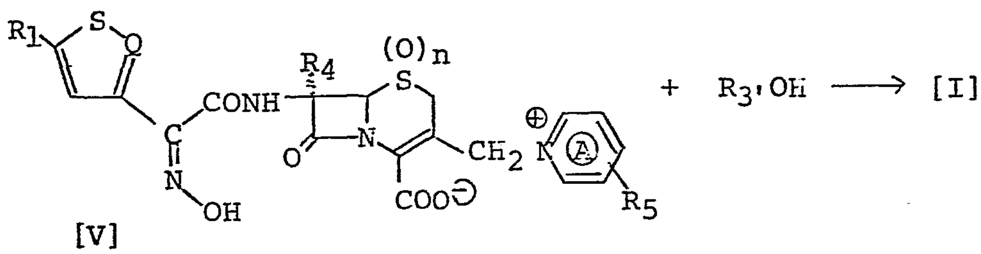

[V]

This method involves a reaction of the hydroxyimino compound [V] with a compound of the formula $R_3'OH$ or its reactive derivative to synthesize the compound [I], and is a well-known etherification reaction. $R_3'$ is a hydrocarbon residue which may be substituted, and as such a hydrocarbon residue, there can apply here those mentioned hereinbefore for $R_3$ as the hydrocarbon residue which may be substituted. $R_3'OH$ is used as such or in the form of its reactive derivative. The reactive derivative of $R_3'OH$ denotes derivatives of $R_3'OH$ having a group eliminating together with the hydrogen atom of the compound [V] or compounds of the formula $R_3'Y$. With reference to the above, the group Y eliminating together with the hydrogen atom is a halogen atom or a sulfo, mono-substituted sulfonyloxy group, etc. The halogen atom includes chlorine, bromine, iodine, etc. The mono-substituted sulfonyloxy group includes $C_{1-4}$ alkylsulfonyloxy and $C_{6-10}$ arylsulfonyloxy groups, such as methanesulfonyloxy ethanesulfonyloxy, benzensulfonyloxy and p-toluenesulfonyloxy, and the like. In cases in which the $C_{1-4}$ alkyl ether derivatives of the compound [V] are produced, particularly, there are used the above-described reactive derivatives as well as $C_{1-4}$ diazoalkanes, such as diazomethane and diazoethane, di-$C_{1-4}$ alkyl sulfates, such as dimethyl sulfate and diethyl sulfate, and the like.

(3-1): when $R_3$'OH is used;

$R_3$'OH is reacted with the compound [V] by using a suitable dehydrating agent to synthesize the compound [I]. The dehydrating agent to be used for such a purpose includes phosphorus oxychloride, thionyl chloride, dialkyl azodicarboxylate (usually used in the presence of phosphine or N,N-dicyclohexylcarbodiimide, etc., and preferably diethyl azodicarboxylate in the presence of triphenylphosphine. The reaction in which diethyl azodicarboxylate is used in the presence of triphenylphosphine usually in an anhydrous solvent such as ethers or hydrocarbons menthioned above. $R_3$'OH, diethyl azodicarboxylate and triphenylphosphine are all used in quantities of 1 to 1.5 moles per mole of the compound [V], respectively. The reaction requires 1 to 4 days in the temperature range of 0 to 50°C.

(3-2): when $R_3$'Y is used.

The reaction between $R_3$'Y and the compound [V] is a usual etherification reaction, and it carried out in a solvent. As the solvent, there may be mentioned solvents, or solvent mixtures, such as ethers, esters, halogenated hydrocarbons, hydrocarbons, amides, ketones, nitriles, alcohols and water, as described under the item of the production method (1), and preferable are solvent mixtures (e.g., aqueous methanol, aqueous ethanol, aqueous acetone, aqueous dimethyl sulfoxide, etc.) of solvents miscible with water and water. This reaction can also be allowed to proceed smoothly in the presence of a suitable base. Such a base includes inorganic bases, such as alkali metal salts being exemplified by sodium carbonate, sodium hydrogencarbonate and potassium carbonate and alkali metal hydroxides being exemplified by sodium hydroxide and potassium hydroxide. Also, this reaction may be conducted in a buffer of pH 7.5 to 8.5. The numbers of moles of the reagent $R_3$'Y and base to be used per mole of the starting compound [V] are 1 to 5 and 1 to 10, preferably 1 to 3 and 1 to 5, respectively. The reaction temperature ranges from -30 to 100°C, preferably from 0 to 80°C. The reaction time is from 10 minutes to 15 hours, preferably from 30 minutes to 5 hours.

(3-3): when a $C_{1-4}$ diazoalkane is used;

The reaction is normally carried out in a solvent. As the solvent, there are used the above-described ethers, hydrocarbons, etc. When the compound [V] is dissolved in a solvent and a solution of a diazoalkane compound is then added, the reaction proceeds. The reagent is used in an amount of 1 to 10 moles per mole of the compound [V], preferably 1 to 5 moles. The reaction is carried out at a relatively low temperature, namely -50 to 20°C, preferably -30 to 0°C. The reaction time is from 1 minute to 5 hours, preferably from 10 minutes to 1 hour.

(3-4): when a di-$C_{1-4}$ alkyl sulfate is used;

The reaction is normally conducted in water or a solvent mixture of a solvent miscible with water and water. As the solvent mixture, there may also be mentioned the aqueous solvents as described under the item (3-2). This reaction is carried out in the presence of an alkali metal hydroxide, such as sodium hydroxide and potassium hydroxide. The reagent is used in an amount of 0.5 to 10 moles per mole of the compound [V], preferably 1 to 2 moles. The reaction temperature is in the range of 20 to 100°C, preferably 50 to 100°C. The reaction time is from 10 minutes to 5 hours, preferably from 30 minutes to 3 hours.

Production method (4):

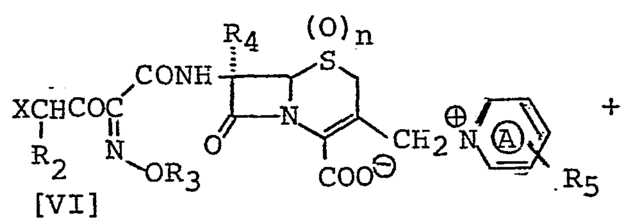

+ $R_1C(=S)NH_2$ ⟶ [I](Q=C-$R_2$)

[VI]

This method involves a reaction of the cephem compound [VI] with a thiourea or thiourea derivative of the formula $R_1C(=S)NH_2$ to synthesize the objective compound [I] (Q=C-R2). The compound [VI] is used in the free form or as a salt or ester. X in the compound [VI] is a halogen atom such as chlorine, bromine, iodine or a group of the formula $R_6SO_2O$ wherein $R_6$ is a $C_{1-4}$ alkyl group, phenyl group or phenyl substituted with a $C_{1-4}$ alkyl, $C_{1-6}$ alkoxy, halogen atom or nitro group. As the $C_{1-4}$ alkyl group, there may be mentioned methyl, ethyl, propyl etc. As the substituted phenyl group, there may be mentioned for example p-methylphenyl, p-methoxyphenyl, p-chlorophenyl, p-nitrophenyl, etc. As the salt of [VI], there can also

apply here the salts (inorganic base salts, ammonium salt , organic base salts, inorganic acid addition salts, organic acid addition salts, etc.) of the compound [II] as described under the item of the production method (1). As the ester of [VI], there can also apply here the esters ($C_{1-4}$ alkyl esters, $C_{2-4}$ alkenyl esters, $C_{3-6}$ cycloalkyl esters, $C_{3-6}$ cycloalkyl-$C_{1-4}$ alkyl esters, $C_{6-10}$ aryl esters, $C_{7-12}$ aralkyl esters, $C_{1-5}$ alkanoyloxymethyl esters, $C_{1-5}$ alkanoyloxyethyl esters and those esters which are further substituted) of the compound [II] as described under the item of the production method (1), as well. The starting compound [VI] can be produced from a compound of the formula

XCHCOC-COOH
$R_2$ N
$OR_3$

wherein the symbols are as defined hereinbefore or its reactive derivative and the above-described 7-amino compound [II] or its salt or ester by using the method similar to the one as described under the production method (1). The compound of the formula

XCHCOC-COOH
$R_2$ N
$OR_3$

or its reactive derivative can be readily produced by the *per se* known method or methods similar thereto. The reaction of the compound [VI] with $R_1C(=S)NH_2$ is normally carried out in a solvent. As the solvent, there are used ethers, such as dioxane, tetrahydrofuran and diethyl ether, alcohols, such as methanol, ethanol and propanol, amides, such as dimethylformamide and dimethylacetamide, and the like. The amount of thiourea or its derivative $R_1C(=S)NH_2$ to be used is normally 1 to 5 moles per mole of the compound [VI], preferably 1 to 3 moles. The reaction is carried out in the temperature range of 0 to 100°C, preferably 20 to 60°C. The reaction time is normally from 30 minutes to 15 hours, preferably from 1 to 5 hours.

In the above-described production methods (1) to (4), the compound [I] is in some instances obtained in the form of a mixture of the *syn* [Z]- and *anti* [E]-isomers. In order to separate the desired *syn* isomer from the mixture, the *per*

*se* known method or methods similar thereto are applied. Such methods include fractionation methods utilizing the difference in solubility, crystallinity, etc., separation by means of chromatography, separation method utilizing the difference in rate of hydrolysis of ester derivatives, and the like.

Method of removing the protective group: As mentioned hereinbefore, the amino protecting groups have been fully studied in the fields of β-lactam and peptide synthesis, and the method of their protection has been already established. The method of removing the amino-protecting groups has been likewise established and, in the present invention, the removal of the protective groups can be effected by utilizing the conventional art as such. For example, monohalogenoacetyl groups (e.g., chloroacetyl, bromoacetyl, etc.), alkoxycarbonyl groups (e.g., methoxycarbonyl, ethoxycarbonyl, tert-butoxycarbonyl, etc.), aralkyloxycarbonyl groups (e.g., benzyloxycarbonyl, p-methylbenzyloxycarbonyl, p-nitrobenzyloxycarbonyl, etc.) and 2,2,2-trichloroethoxycarbonyl group can be removed by thiourea, acids (e.g., hydrochloric acid, etc.), catalytic reduction and zinc and acids (e.g., acetic acid, etc.), respectively. On the other hand, when the compound [I] as a synthetic intermediate is esterified, the ester group can be removed by the *per se* known method or methods similar thereto. For example, 2-methylsulfonylethyl ester, aralkyl esters (e.g., benzyl ester, p-methoxybenzyl ester, p-nitrobenzyl ester,etc.), 2,2,2-trichloroethyl ester and silyl esters (e.g., trimethylsilyl ester, tert-butyldimethylsilyl ester, etc.) can be removed by alkali, acids (e.g., trifluoroacetic acid, etc.) or catalytic reduction, zinc and acids (e.g., acetic acid, etc.) and water alone, respectively.

Method of purifying the compound [I]: The compound [I] formed in the reaction mixture in accordance with the various production methods as described in detail under (1) to (4), followed by the above-described procedure of removing the protective group, if necessary, can be isolated and purified by the known procedures, such as extraction method,

column chromatography, precipitation method and recrystallization method. On the other hand, the isolated compound [I] can also be converted into the desired, physiologically accetable salts or metabolically unstable, nontoxic esters by the known method.

The sulfoxide (n = 1) of the cephem compound [I] is obtained by an oxidation reaction of the compound [I] (n = 0). Such an oxidation reaction is a well-known reaction. The oxidizing agent suitable for the oxidation of the sulfur atom in the cephem ring includes, for example, oxygen, peracids, hydroperoxides, hydrogen peroxide, etc., whereby the peracids can also be produced by the in situ mixing of acid and peroxide. As such peroxides, use is often made of peracetic acid, perbenzoic acid or p-chloroperbenzoic acid. The reaction is normally carried out in a solvent.
The solvent which is useful for the reaction includes ethers, such as dioxane and tetrahydrofuran , halogenated hydrocarbons, such as dichloromethane, chloroform and chlorobenzene, organic acids, such as formic acid, acetic acid and trifluoroacetic acid, and amides, such as diemthylformamide and dimethylacetamide. The reaction is conducted at temperatures in the range of -20 to 80°C, but at as low temperatures as possible, preferably -20 to 20°C. In the oxidation of the compound of the formula [I] wherein n = 0, it has been generally known that the sulfoxide having the S-stereochemical configuration is mainly formed. The R- and S-sulfoxides are separated from each other by means of their different solubilities and their varying transfer rates in chromatographic separation. The above-described oxidation reaction for the purpose of obtaining the sulfoxide may be carried out in advance of and after, the above-described reactions as mentioned under (1) to (4).

The compounds [I] of the present invention, like the known penicillins and cephalosporins, can be administered parenterally or orally in the forms of injectable solutions, capsules, tablets and granules. With reference to their dosages, they may desirably be administered in doses of 0.5 to 80 mg/day per kg of body weight of human being or animals infected with the above-described pathogenic bacteria,

more preferably 1 to 20 mg/day, being divided into three to four times a day. In cases in which they are used as an injectable solution, such a carrier as distilled water and physiological saline is utilized, and when they are used as a capsule, powder, granule and tablet, they are employed as admixtures with the known, pharmacologically accetable excipients (e.g., starch, lactose, sucrose, calcium carbonate, calcium phosphate, etc.), binding agents (e.g., starch, gum arabic, carboxymethylcellulose, hydroxypropylcellulose, crystalline cellulose, etc.), lubricants (e.g., magnesium stearate, talc, etc.) and disintegrating agents (e.g., carboxymethylcalcium, talc, etc.).

A pharmacutical composition containing the compound [I] or a salt or ester thereof is made by a known procedure. The composition is usually produced by mixing at least one of the compounds [I] or their salts or esters with the above carriers or excipients. The ratio of the compound [I] to the whole composition is usually 5 to 100% (w/w), preferably 20 to 100% (w/w) in solid composition such as capsules, tablets and granules, 5 to 30% (w/w) in liquid composition such as injections etc.

The compound [I] or its physiologically or pharmaceutically acceptable salt or ester is preferably administered as an injection for example for combatting against urinary tract infections caused by Escherichia coli. In this case, the dosage amount is in the range of from 1 to 20 mg/kg in 3 to 4 divided doses relative to one kilogram of the body weight of adult human. The injection is easily prepared by dissolving or suspending the compound [I], its salt or ester into physiological saline.

The reference examples and examples are described below to illustrate furthermore the present invention, but it is to be understood that these examples are given for illustrative purposes only and shall not limit the present invention, and that various changes and modifications may be made in the invention without departing from the scope thereof.

In the reference examples and examples, elution in column chromatography was effected under observation by means of TLC (Thin Layer Chromatography). In the TLC observation, $BOF_{254}$ produced by Merck Co. was used as a TLC plate, while the solvent utilized as a eluting solvent in column chromatography was employed as a developing solvent, and a UV detector was adopted for detection. The silica gel for column chromatographic use employed was Kiesel Guhr 60 (230 to 400 mesh) produced by Merck Co., as well. "Sephadex" is produced by Pharmacia Fine Chemicals, and XAD-2 resin is produced by Rohm & Haas Co. The NMR spectra were taken with an XL-100A (100 MHZ), EM 390 (90 MHz) or $T_{60}$ (60 MHz) type of spectrometer using tetramethylsilane as internal or external standard, whereby the total δ value was expressed in ppm. The figures as parenthesized in the solvent mixtures denote a mixing ratio on a volume basis for each of the solvents. % in the solutions designates a number of g in 100 mℓ of the solution. The symbols in the reference examples and examples have the following

meanings:

s : Singlet
d : Doublet
t : Triplet
q : Quartet
ABq : AB type quartet
d.d : Double doublet
m : Multiplet
br. : Broad
J : Coupling constant
Hz : Herz
mg : Milligram
g : Gram
mℓ : Milliliter
ℓ : Liter
% : Percent
DMSO: Dimethylsufloxide

Reference Example 1

7β-[2-(2-Chloroacetamidothiazol-4-yl)-2(Z)-methoxyiminoacetamido]-3-(3-oxobutyryloxymethyl)-3-cephem-4-carboxylic acid

In a mixture consisting of 500 mℓ of tetrahydrofuran and 500 mℓ of water is suspended 157 g of 7β-amino-3-(3-oxobutyryloxymethyl)-3-cephem-4-carboxylic acid. To the suspension, 141 g of sodium bicarbonate is added little by little with stirring. One hundred and fifty grams of 2-(2-chloroacetamido-thiazol-4-yl)-2(Z)-methoxyiminoacetyl chloride hydrochloride is added to the mixture at 5°C over the period of 20 minutes with stirring and the reaction mixture is stirred at the same temperature for another 1 hour. After completion of the reaction, the reaction mixture is adjusted to pH 3.0 and extracted with two 1ℓ portions of a mixture of ethyl acetate-tetrahydrofuran (1:1). The extract is dried over anhydrous magnesium sulfate, and the solvent is distilled off under reduced pressure. The resulting colorless

powder is washed with 200 mℓ of ethyl acetate and collected by filtration to give 253 g of the titled compound.

Elemental analysis, for $C_{20}H_{20}ClN_5O_9S_2$:

Calcd.(%): C, 41.85; H, 3.51; N, 12.20.

Found (%): C, 41.39; H, 3.57; N, 11.94.

IR spectrum $\nu_{max}^{KBr}$ $cm^{-1}$: 1780, 1740, 1700, 1655, 1540, 1410.

NMR spectrum ($d_6$-DMSO) δ: 2.20(3H,s), 3.45 and 3.68(2H,ABq, J=18Hz), 3.65(2H,s), 3.92(3H,s), 4.38(2H,s), 4.79 and 5.09 (2H,ABq,J=13Hz), 5.18(1H,d,J=5Hz), 5.85(1H,d.d,J=5Hz and 8Hz), 7.44(1H,s), 9.66(1H,d,J=8Hz), 12.85(1H,br.s).

Reference Example 2

7β-[2-(2-Aminothiazol-4-yl)-2(Z)-methoxyiminoacetamido]-3-(3-oxobutyryloxymethyl)-3-cephem-4-carboxylic acid

In 500 mℓ of a mixture of tetrahydrofuran-water (1:1) are dissolved 150 g of 7β-2-[2-(chloroacetamido-thiazol-4-yl)-2(Z)-methoxyiminoacetamido]-3-(3-oxobutyryl-oxymethyl)-3-cephem-4-carboxylic acid and 51 g of sodium N-methyldithiocarbamate and the mixture is stirred at 20°C for 3 hours. Two hundred mℓ of ethyl acetate is added to the reaction mixture and the organic layer is removed. The aqueous layer is adjusted to pH 4 by adding 10 % hydrochloric acid, and an oily material which separates out is extracted with 1 ℓ of a mixture of tetrahydrofuran-ethyl acetate (1:1). The aqueous layer is further extracted with 200 mℓ of n-butanol. The extracts are combined and dried over anhydrous sodium sulfate, and the solvent is distilled off under reduced pressure. The residue is treated with 200 mℓ of ethyl acetate and the crystals which separate out are collected by filtration to give 90 g of the titled compound.

Elemental analysis, for $C_{18}H_{19}N_5O_8S_2$:

Calcd.(%): C, 42.19; H, 4.30; N, 13.55.

Found (%): C, 41.94; H, 4.11; N, 13.59.

IR spectrum $\nu_{max}^{KBr}$ $cm^{-1}$: 1770, 1710, 1620, 1520.

NMR spectrum ($d_6$-DMSO)δ: 2.20(3H,s), 3.43 and 3.65(2H,ABq, J=18Hz), 3.63(2H,s), 3.86(3H,s), 4.78 and 5.06(2H,ABq,J=13Hz),

5.14(1H,d,J=5Hz), 5.79(1H,d.d,J=5Hz and 8Hz), 6.73(1H,s), 7.17(2H,br.), 9.56(1H,d,J=8Hz).

Reference Example 3

7β-[2-(5-Amino-1,2,4-thiadiazol-3-yl)-2(Z)-ethoxyiminoacetamido]-3-(3-oxobutyryloxymethyl)-3-cephem-4-carboxylic acid.

In 200 ml of dichloromethane is suspended 11g of 7β-amino-3-(3-oxobutyryloxymethyl)-3-cephem-4-carboxylic acid. To the suspension is added 14 g of bistrimethylsilylacetamide and the mixture is stirred at room temperatrue until complete dissolution and cooled in and ice-water bath. To this solution, 14 g of 2-(5-amino-1,2,4-thiadiazol-3-yl)-2(Z)-ethoxyiminoacetyl chloride is added and the mixture is stirred for a while, to which 6 g of dimethylacetamide is added. The whole mixture is stirred with ice-cooling for 60 minutes. The dichloromethane is evaporated off and the residue is dissolved in methyl ethyl ketone. The solution is washed with water and dried. The solvent is then evaporated off and diethyl ether is added to the residue to give a fine precipitate, which is collected by filtration, giving 12.5 g of the titled compound.

IR spectrum $\nu_{max}^{KBr}$ $cm^{-1}$: 3300, 3000, 1780, 1720, 1620, 1520, 1410, 1260, 1150, 1040.

NMR spectrum ($d_6$-DMSO) δ: 1.25(3H,t,J=7Hz), 2.18(3H,s), 3.41 and 3.63(2H,ABq,J=18Hz), 3.62(2H,s), 4.18(2H,q,J=7Hz), 4.76 and 5.06(2H,ABq,J=13Hz), 5.14(1H,d,J=4.8Hz), 5.82 (1H,d.d,J=8Hz and 4.8Hz), 8.00(2H,br.), 9.48(1H,d,J=8Hz).

Example 1

7β-[2-(2-Aminothiazol-4-yl)-2(Z)-methoxyiminoacetamido]-3-[[4-(1,2,4-oxadiazol-3-yl)pyridinium-1-yl]methyl]-3-cephem-4-carboxylate

In 20 mℓ of a mixture of acetonitrile and water (1:1) are dissolved 2.0 g of 7 -[2-(2-aminothiazol-4-yl)-2(Z)-methoxyiminoacetamido]-3-(3-oxobutyryloxymethyl)-3-cephem-4-carboxylic acid, 2.0 g of 4-(1,2,4-oxadiazol-3-yl)-pyridine and 2.0 g of potassium iodide and the mixture is stirred at 70°C for 2 hours. The solvent is distilled off under reduced pressure, and the residue is triturated with acetonitrile. Insoluble substance is suspended in 20 ml of water and the suspension is adjusted to pH 8.0 by adding sodium bicarbonate and chromatographed on a column of XAD-2 resin, elution being carried out with 10 % aqueous methanol. Fractions containing the desired compound are combined and concentrated under reduced pressure. The residue is lyophilized and a solid material obtained is dissolved in 5 mℓ of water and chromatographed on a column of silica gel. Elution is effected with a mixture of acetonitrile and water (4:1) and fractions containing the desired compound are combined and concentrated under reduced pressure, and the residue is lyophilized to give 0.23 g of the titled compound.

Elemental analysis, for $C_{21}H_{18}N_8O_6S_2 \cdot 2H_2O$:

Calcd. (%): C, 42.43; H, 3.73; N, 18.85.

Found (%) : C, 42.29; H, 3.58; N, 18.98.

IR spectrum $\nu_{max}^{KBr} cm^{-1}$: 1770, 1660, 1620, 1530, 1040.

NMR spectrum ($d_6$-DMSO) δ: 3.10 and 3.50(2H,ABq,J=18Hz), 3.80 (3H,s), 5.06(1H,d,J=4.5Hz), 5.30-5.90(3H,m), 6.66(1H,s), 7.20(2H,br.s), 7.70(1H,s), 8.35(2H,d,J=6Hz), 9.40(2H,d, J=6Hz), 9.60(1H,d,J=9Hz).

Example 2

7β-[2-(2-Aminothiazol-4-yl)-2(Z)-methoxyiminoacetamido-3-[[3-(1,2,4-oxadiazol-3-yl)pyridinium-1-yl]methyl]-3-cephem-4-carboxylate

7β-[2-(2-Aminothiazol-4-yl)-2(Z)-methoxyimino-actamido]-3-(3-oxobutyryloxymethyl)-3-cephem-4-carboxylic acid, 3-(1,2,4-oxadiazol-3-yl)pyridine and potassium iodide are reacted in the same procedure as Example 1 to give the titled compound.

Elemental analysis, for $C_{21}H_{18}N_8O_6S_2 \cdot 3H_2O$:

Calcd.(%): C, 42.28; H, 4.06; N, 18.78.

Found (%): C, 42.09; H, 4.03; N, 18.75.

IR spectrum $\nu_{max}^{KBr}$ $cm^{-1}$: 1780, 1670, 1620, 1530, 1390, 1040.

NMR spectrum ($d_6$-DMSO)δ : 3.20 and 3.60(2H,ABq,J=18Hz), 3.85 (3H,s), 5.15(1H,d,J=4.5Hz), 5.30(1H,ABq,J=12Hz), 5.60-5.90 (3H,m), 6.80(1H,s), 7.30(2H,br.s), 8.0-8.33(2H,m), 8.10-8.43(1H,m), 8.70-9.10(1H,m), 9.30-9.85(3H,m).

## Example 3

7β-[2-(2-Aminothiazol -4-yl)-2(Z)-methoxyiminoacetamido]-3-[[3-(imidazol-2-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 3-(imidazol-2-yl)pyridine and potassium iodide are reacted in the same procedure as Example 1 to give the titled compound.

Elemental analysis, for $C_{22}H_{20}N_8O_5S_2 \cdot 7/2H_2O$:

Calcd.(%): C, 43.78; H, 4.51; N, 18.56.

Found (%): C, 44.03; H, 4.17; N, 18.44.

IR spectrum $\nu_{max}^{KBr}$ $cm^{-1}$: 1780, 1670, 1620, 1535.

NMR spectrum ($d_6$-DMSO)δ : 3.20 and 3.58(2H,ABq,J=18Hz), 3.79 (3H,s), 5.07(1H,d,J=4.5Hz), 5.31 and 5.80(2H,ABq,J=13Hz), 5.55-5.80(1H,m), 6.68(1H,s), 7.14(2H,br.s), 7.29(2H,s), 8.0-8.35(1H,m), 8.9-9.15(1H,m), 9.4-9.6(2H,m), 9.94(1H,br.s).

## Example 4

7β-[2-(2-Aminothiazol-4-yl)-2(Z)-methoxyiminoacetamido]-3-[[4-(imidazol-2-yl)pyridinium-1-yl]methyl]-3-cephem-4-carboxylate

7β-[2(2-Aminothiazol-4-yl)-2(Z)-methoxyimino-acetamido]-3-(3-oxobutyryloxymethyl)-3-cephem-4-carboxylic

acid, 4-(imidazol-2-yl)pyridine and potassium iodide are reacted in the same procedure as Example 1 to give the titled compound.

Elemental analysis, for $C_{22}H_{20}N_8O_5S_2 \cdot 4H_2O$:

Calcd.(%): C, 43.13; H, 4.61; N, 18.29.

Found (%): C, 42.97; H, 4.12; N, 17.83.

IR spectrum $\nu_{max}^{KBr}cm^{-1}$: 1775, 1670, 1650, 1610, 1535.

NMR spectrum ($d_6$-DMSO) δ: 3.15 and 3.60(2H,ABq,J=18Hz), 3.77 (3H,s), 5.10(1H,d,J=4.5Hz), 5.4-5.8(3H,m), 6.68(1H,s), 7.13 (2H,br.s), 7.47(2H,s), 8.58(2H,d,J=6Hz), 9.37(2H,d,J=6Hz), 9.51(1H,d,J=8Hz).

Example 5

7β-[2-(2-Aminothiazol-4-yl)-2(Z)-methoxyiminoacetamido]-3-[[3-(5-methyl-1,2,4-oxadiazol-3-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 3-(5-methyl-1,2,4-oxazol-3-yl)pyridine and potassium iodide are reacted in the same procedure as Example 1 to give the titled compound.

Elemental analysis, for $C_{22}H_{20}N_8O_6S_2 \cdot 3H_2O$:

Calcd.(%): C, 43.28; H, 4.29; N, 18.35.

Found (%): C, 43.18; H, 3.96; N, 18.40.

IR spectrum $\nu_{max}^{KBr}cm^{-1}$: 1780, 1665, 1620, 1530, 1390, 1050.

NMR spectrum ($d_6$-DMSO) δ: 2.70(3H,s), 3.20 and 3.60(2H,ABq,J=18Hz), 3.80(3H,s), 5.10(1H,d,J=4.5Hz), 5.20-5.80(3H,m), 6.70 (1H,s), 7.20(2H,br.s), 8.20-8.50(1H,m), 8.85-9.20(1H,m), 9.40-9.80(2H,m), 10.1(1H,s).

Example 6

7β-[2-(2-Aminothiazol-4-yl)-2(Z)-methoxyiminoacetamido]-3-[[4-(5-methyl-1,2,4-oxadiazol-3-yl)pyridinium-1-yl]methyl]-3-cephem-4-carboxylate.

7β-[2-(2-Aminothiazol-4-yl)-2(Z)-methoxyimino-acetamido]-3-(3-oxobutyryloxymethyl)-3-cephem-4-carboxylic acid, 4-(5-methyl-1,2,4-oxadiazol-3-yl)pyridine and potassium iodide are reacted in the same procedure as Example 1 to give the titled compound.

to give the titled compound.

Elemental analysis, for $C_{22}H_{20}N_8O_6S_2 \cdot 9/2H_2O$:

Calcd.(%): C, 41.45; H, 4.58; N, 17.57.

Found (%): C, 41.54; H, 4.62; N, 17.95.

IR spectrum $\nu_{max}^{KBr} cm^{-1}$: 1775, 1670, 1620, 1530, 1390, 1040.

NMR spectrum ($d_6$-DMSO-$D_2O$)$\delta$ : 2.90(3H,s).3.30-3.80(2H,ABq, J=18Hz), 5.30(1H,d,J=4.5Hz), 5.40-6.00(3H,m), 6.90(1H,s), 8.80(2H,d,J=6Hz), 9.63(2H,d,J=6Hz).

Example 7

7β-[2-(2-Aminothiazol-4-yl)-2(Z)-methoxyiminoacetamido]-3-[[4-(5-isopropyl-1,2,4-oxadiazol -3-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(5-isopropyl-1,2,4-oxadiazol-3-yl)pyridine and potassium iodide are reacted in the same procedure as Example 1 to give the titled compound.

Elemental analysis, for $C_{24}H_{24}N_8O_6S_2 \cdot 4H_2O$:

Calcd.(%): C, 43.90; H, 4.91; N, 17.07.

Found (%): C, 43.57; H, 5.25; N, 16.84.

IR spectrum $\nu_{max}^{KBr} cm^{-1}$: 1780, 1680, 1610, 1530, 1380, 1040.

NMR spectrum ($d_6$-DMSO)$\delta$ : 1.45(6H,d,J=8Hz), 3.15(1H,ABq,J=18Hz), 3.25-3.70(3H,m), 3.80(3H,s), 5.13(1H,d,J=5Hz), 5.33 and 5.86(2H,ABq,J=12Hz), 5.73(1H,d.d,J=8Hz and 4.5Hz), 6.70 (1H,s), 7.25(2H,br.s), 8.70(2H,d,J=6Hz), 9.53-9.80(3H,m).

Example 8

7β-[2-(2-Aminothiazol -4-yl)-2(Z)-methoxyiminoacetamido]-3-[[4-(5-hydroxy-1,2,4-oxadiazol -3-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(5-hydroxy-1,2,4-oxadiazol-3-yl)pyridine and potassium iodide are reacted in the same procedure as Example 1 to give the titled compound.

Elemental analysis, for $C_{21}H_{18}N_8O_7S_2 \cdot 3H_2O$:

Calcd.(%): C, 41.20; H, 3.95; N, 18.30.

Found (%): C, 40.96; H, 4.27; N, 18.11.

IR spectrum $\nu_{max}^{KBr}cm^{-1}$: 1760, 1630, 1520, 1250, 1030

NMR spectrum ($d_6$-DMSO) $\delta$: 3.15 and 3.56(2H,ABq,J=18Hz), 3.80 (3H,s), 5.10(1H,d,J=4.5Hz), 5.20-5.90(3H,m), 6.70(1H,s), 7.20(2H,br.s), 7.53(1H,s), 8.50(2H,d,J=6Hz), 9.60(2H,d,J=6Hz), 9.40-9.56(1H,m).

Example 9

7β-[2-(2-Aminothiazol-4-yl)-2(Z)-methoxyiminoacetamido]-3-[[4-(1,3,4-oxadiazol-2-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(1,3,4-oxadiazol-2-yl)pyridine and potassium iodide are reacted in the same procedure as Example 1 to give the titled compound.

Elemental analysis, for $C_{21}H_{18}N_8O_6S_2 \cdot 3H_2O$:

Calcd.(%): C, 42.28; H, 4.06; N, 18.78.

Found (%): C, 42.51; H, 4.40; N, 18.56.

IR spectrum $\nu_{max}^{KBr}cm^{-1}$: 1780, 1670, 1620, 1530, 1040.

NMR spectrum ($d_6$-DMSO) $\delta$: 3.20(1H,ABq,J=18Hz), 3.60-3.90 (4H,m), 5.10(1H,d,J=5Hz), 5.35(1H,ABq,J=12Hz), 5.60-5.90 (3H,m), 6.70(1H,s), 7.20(2H,br.s), 8.65(2H,d,J=6Hz), 9.50 (1H,s), 9.60-9.80(3H,m).

Example 10

7β-[2-(2-Aminothiazol-4-yl)-2(Z)-methoxyiminoacetamido]-3-[[3-(1,3,4-oxadiazol-2-yl)pyridinium-1-yl]methyl]-3-cephem-4-carboxylate.

7β-[2-(2-Aminothiazol-4-yl)-2(Z)-methoxyimino-acetamido]-3-(3-oxobutyryloxymethyl)-3-cephem-4-carboxylic acid, 3-(1,3,4-oxadiazol-2-yl)pyridine and potassium iodide are reacted in the same procedure as Example 1 to give the titled compound.

Elemental analysis, for $C_{21}H_{18}N_8O_6S_2 \cdot 3H_2O$:

Calcd.(%): C, 42.28; H, 4.06; N, 18.78.

Found (%): C, 41.95; H, 4.36; N, 18.49.

IR spectrum $\nu_{max}^{KBr}cm^{-1}$: 1780, 1620, 1530, 1040.

NMR spectrum ($d_6$-DMSO) $\delta$: 3.20 and 3.50(2H,ABq,J=18Hz), 3.80

(3H,s), 5.51(1H,d,J=4.5Hz), 5.30 and 5.80(2H,ABq,J=12Hz), 5.55-5.70(1H,m), 6.70(1H,s), 7.20(2H,br.s), 8.30-8.50 (2H,m), 8.90-9.20(1H,m), 9.50(1H,s), 9.75(1H,d,J=8Hz), 10.20(1H,s).

Example 11

7β-[2-(2-Aminothiazol-4-yl)-2(Z)-methoxyiminoacetamido]-3-[[4-(5-methyl-1,3,4-oxadiazol-2-yl)pyridinium-1-yl]methyl]-3-cephem-4-carboxylate

7β-[2(2-Aminothiazol-4-yl)-2(Z)-methoxyimino-acetamido]-3-(3-oxobutyryloxymethyl)-3-cephem-4-carboxylic acid, 4-(5-methyl-1,3,4-oxadiazol-2-yl)pyridine and potassium iodide are reacted in the same procedure as Example 1 to give the titled compound.

Elemental analysis, for $C_{22}H_{20}N_8O_6S_2 \cdot 3H_2O$:

Calcd.(%): C, 43.28; H, 4.29; N, 18.35.

Found (%): C, 43.05; H, 4.50; N, 18.27.

IR spectrum $\nu_{max}^{KBr} cm^{-1}$: 1780, 1660, 1620, 1540, 1040

NMR spectrum ($d_6$-DMSO) δ: 2.60(3H,s), 3.20(1H,ABq,J=18Hz), 3.80(3H,s), 5.10(1H,d,J=4.5Hz), 5.30 and 5.80(2H,ABq,J=14Hz), 5.73(1H,d.d,J=8Hz and 5Hz), 6.65(1H,s), 7.20(2H,br.s), 8.60(2H,d,J=6Hz), 9.40-9.73(3H,m).

Example 12

7β-[2-(2-Aminothiazol-4-yl)-2(Z)-methoxyiminoacetamido]-3-[[4-(isoxazol-5-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(isoxazol-5-yl)pyridine and potassium iodide are reacted in the same procedure as Example 1 to give the titled compound.

Elemental analysis, for $C_{22}H_{19}N_7O_6S_2 \cdot 3H_2O$:

Calcd.(%): C, 44.37; H, 4.23; N, 16.47.

Found (%): C, 44.15; H, 4.47; N, 16.19.

IR spectrum $\nu_{max}^{KBr} cm^{-1}$: 1770, 1670, 1610, 1530, 1040.

NMR spectrum ($d_6$-DMSO) δ: 3.20-3.60(2H,m), 3.80(3H,s), 5.13 (1H,d,J=4.5Hz), 5.30-5.90(3H,m), 6.70(3H,s), 7.25(2H,br.s),

7.60(1H,s), 8.30-8.43(3H,m), 9.30-9.60(3H,m).

Example 13

7β-[2-(2-Aminothiazol-4-yl)-2(Z)-methoxyiminoacetamido]-3-[[3-(isoxazol-5-yl)pyridinium-1-yl]methyl]-3-cephem-4-carboxylate

7β-[2-(2-Aminothiazol-4-yl)-2-(Z)-methoxyimino-acetamido]-3-(3-oxobutyryloxymethyl)-3-cephem-4-carboxylic acid, 3-(isoxazol-5-yl)pyridine and potassium iodide are reacted in the same procedure as Example 1 to give the titled compound.

Elemental analysis, for $C_{22}H_{19}N_7O_6S_2 \cdot 7/2H_2O$:

Calcd.(%): C, 43.71; H, 4.34; N, 16.22.

Found (%): C, 43.59; H, 4.60; N, 15.99.

IR spectrum $\nu_{max}^{KBr} cm^{-1}$: 1780, 1670, 1620, 1520, 1040.

NMR spectrum ($d_6$-DMSO) δ: 3.20-3.60(2H,m), 3.75(3H,s), 5.03 (1H,d,J=4.5Hz), 5.20-5.90(3H,m), 6.70(1H,s), 6.90(1H,s), 7.20(2H,br.s), 7.80(1H,s), 7.90-8.20(1H,m), 8.70-8.90 (1H,m), 9.30-9.65(2H,m), 9.93(1H,s).

Example 14

7β-[2-(2-Aminothiazol-4-yl)-2(Z)-methoxyiminoacetamido]-3-[[4-(pyrazol-3-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(pyrazol-3-yl)pyridine and potassium iodide are reacted in the same procedure as Example 1 to give the titled compound.

Elemental analysis, for $C_{22}H_{20}N_8O_5S_2 \cdot 4H_2O$:

Calcd.(%): C, 43.13; H, 4.61; N, 18.29.

Found (%): C, 42.88; H, 4.81; N, 18.03.

IR spectrum $\nu_{max}^{KBr} cm^{-1}$: 1770, 1640, 1620, 1530, 1350, 1040.

NMR spectrum ($d_6$-DMSO) δ: 3.15 and 3.60(2H,ABq,J=18Hz), 5.10 (1H,d,J=4.5Hz), 5.30-5.85(4H,m), 6.66(1H,s), 7.23(3H,br.s), 8.80(1H,s), 8.50(2H,d,J=6Hz), 9.36(2H,d,J=6Hz), 9.66(1H,d, J=9Hz).

Example 15

7β-[2-(2-Aminothiazol-4-yl)-2(Z)-methoxyiminoacetamido]-3-[[3-(pyrazol-3-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 3-(pyrazol-3-yl)pyridine and potassium iodide are reacted in the same procedure as Example 1 to give the titled compound.

Elemental analysis, for $C_{22}H_{20}N_8O_5S_2 \cdot 4H_2O$:

Calcd.(%): C, 43.13; H, 4.61; N, 18.29.

Found (%): C, 42.90; H, 4.85; N, 18.00.

IR spectrum $\nu_{max}^{KBr} cm^{-1}$: 1780, 1660, 1620, 1530, 1340, 1040.

NMR spectrum ($d_6$-DMSO) δ : 3.20 and 3.50(2H,ABq,J=18Hz), 3.80 (3H,s), 5.13(1H,d,J=4.5Hz), 5.30-5.90(3H,m), 6.70(1H,s), 7.16(2H,br.s), 7.60(1H,s), 8.00-8.30(1H,m), 8.46-8.90 (2H,m), 9.30-9.80(3H,m).

Example 16

7β-[2-(2-Aminothiazol-4-yl)-2(Z)-methoxyiminoacetamido]-3-[[4-(4-methylthiazol-2-yl)pyridinium-1-yl]methyl]-3-cephem-4-carboxylate

7β-[2-(2-Aminothiazol-4-yl)-2(Z)-methoxyimino-acetamido]-3-(3-oxobutyryloxymethyl)-3-cephem-4-carboxylic acid, 4-(4-methylthiazol-2-yl)pyridine and potassium iodide are reacted in the same procedure as Example 1 to give the titled compound.

Elemental analysis, for $C_{23}H_{21}N_7O_5S_3 \cdot 2H_2O$:

Calcd.(%): C, 45.46; H, 4.15; N, 16.13.

Found (%): C, 45.40; H, 3.98; N, 16.15.

IR spectrum $\nu_{max}^{KBr}$ $cm^{-1}$: 1770, 1675, 1630, 1520, 1465, 1380.

NMR spectrum ($d_6$-DMSO-$D_2O$) δ : 2.58(3H,s), 3.25 and 3.69(2H, ABq,J=18Hz), 3.96(3H,s), 5.24(1H,d,J=5Hz), 5.45(1H,ABq,J= 13Hz), 5.82(1H,d,J=5Hz), 6.92(1H,s), 7.73(1H,s), 8.49(2H,d, J=6Hz), 9.06(2H,d,J=6Hz).

Example 17

7β-[2-(2-Aminothiazol-4-yl)-2(Z)-methoxyiminoacetamido]-3-[[3-(pyrazol-3-yl)pyridinium-1-yl]methyl]-3-cephem-4-carboxylate

In 40 mℓ of a mixture of acetone and water (1:1) are dissolved 2.0 g of 7β-[2-(2-aminothiazol-4-yl)-2(Z)-methoxyiminoacetamido]-3-acetoxymethyl-3-cephem-4-carboxylic acid, 2.0 g of 3-(pyrazol-3-yl)pyridine and 2.0 g of potassium iodide, and the mixture is stirred at 70°C for 2 hours. The reaction mixture is concentrated under reduced pressure, and the residue is triturated with 20 ml of ethyl acetate, suspended in 50 mℓ of water and dissolved in it by addition of sodium bicarbonate. The homogeneous solution thus obtained is chromatographed on a column of XAD-2, and elution is effected with 10 % aqueous ethanol. Fractions containing the desired compound are collected, concentrated under reduced pressure and lyophilized to give 0.3 g of a yellowish powder. It was confirmed from the IR and NMR spectra that this product is identical with the compound obtained in Example 15.

Example 18

7β-[2-(2-Aminothiazol-4-yl)-2(Z)-methoxyiminoacetamido]-3-[[4-(4-methylthiazol-2-yl)pyridinium1-yl]methyl]-3-cephem-4-carboxylate

In a mixture consisting of 10 ml of acetonitrile and 10 mℓ of formamide is suspended 2.0 g of 7β-[2-(2-amino-thiazol-4-yl)-2(Z)-methoxyiminoacetamido]-3-hydroxymethyl-3-cephem-4-carboxylic acid. To this suspension 3.5 g of 4-(4-methylthiazol-2-yl)pyridine and 10 ml of a dichloromethane solution of methyl o-phenylenephosphate (2 mmol/ml) are added, successively at -30°C and the mixture is stirred for 2 hours. The solvent is distilled off under reduced pressure. Twenty ml of acetonitrile is added to the residue and an insoluble solid is collected by filtration. This solid material is suspended in 20 mℓ of water, and the suspension is adjusted to pH 8.0 by adding sodium bicarbonate and chromatographed on a column of XAD-2. Elution is effected with 10 % aqueous ethanol. Fractions containing the desired compound are collected and concentrated under reduced pressure and the residue is lyophilized to give 1.0 g of the titled compound. It was confirmed from the IR and NMR spectra that this product is identical with the compound obtained in Example 16.

Example 19

7β-[2-(2-Aminothiazol-4-yl)-2(Z)-methoxyiminoacetamido]-3-[[4-(4-methylthiazol-2-yl)pyridinium-1-yl]methyl]-3-cephem-4-carboxylate

In 20 mℓ of chloroform is suspended 3.0 g of 7β-[2-(2-aminothiazol-4-yl)-2(Z)-methoxyiminoacetamido]-3-acetoxymethyl-3-cephem-4-carboxylic acid. Four ml of N-methyl-N-trimethylsilyltrifluoroacetamide is added to the suspension and the mixture is stirred at 20°C until a homogeneous solution is formed. Then, 3.3 g of trimethylsilyl iodide is added to the solution, and after being stirred at 20°C for 10 minutes, the reaction solution is concentrated under reduced pressure. Nine ml of acetonitrile is added to the residue, and 0.8 ml of tetrahydrofuran is further added to the mixture, followed by stirring for 30 minutes. Subsequently, 3.0 g of 4-(4-methylthiazol-2-yl)pyridine is added to the mixture, followed by stirring at room temperature for 3 hours. The reaction mixture is cooled to 0°C, and a solid material which separates out upon addition of 1 mℓ of water is collected by filtration. This material is chromatographed on a column of silica gel and eluted with a mixture of acetonitrile and water (4:1). Fractions containing the desired compound are collected, concentrated under reduced pressure and the residue is lyophilized to give 0.2 g of the titled compound.

Example 20

7β-[2-(2-Aminothiazol-4-yl)-2(Z)-methoxyiminoacetamido]-3-[[4-(4-methylthiazol-2-yl)pyridinium-1-yl]methyl]-3-cephem-4-carboxylate

a) In 50 mℓ of a mixture of acetonitrile and water (1:1) are dissolved 5.0 g of 7β-amino-3-(3-oxobutyryloxymethyl)-3-cephem-4-carboxylic acid, 5.0 g of 4-(4-methylthiazol-2-yl)pyridine and 5.0 g of potassium iodide, and the mixture is stirred at 70°C for 2 hours. The solvent is distilled off under reduced pressure. The residue is triturated with 30 mℓ of acetonitrile and the solid material is collected by filtration. This material is dissolved in 30 mℓ of

water, and the solution is chromatographed on a column of XAD-2. Elution is effected with water. Fractions containing the desired compound are collected and concentrated under reduced pressure, and the residue lyophilized to give 1.0 g of 7β-amino-3-[[4-(4-methylthiazol-2-yl)pyridinium-1-yl]methyl]-3-cephem-4-carboxylate.

b) In 20 mℓ of dimethylformamide are dissolved 0.64 g of 2-(2-aminothiazol-4-yl)-2(Z)-methoxyiminoacetic acid and 0.5 g of 1-hydroxybenzotriazole. To the solution 0.7 g of dicyclohexylcarbodiimide is added and the mixture is stirred at 20°C for 2 hours. Insoluble substance which separates out is filtered off and the filtrate is added to a solution of 1.0 g of 7β-amino-3-[[4-(4-methylthiazol-2-yl)-pyridinium-1-yl]methyl]-3-cephem-4-carboxylate obtained in a) in 20 mℓ of dimethylformamide. The mixture is stirred at 20°C for 3 hours. After 200 mℓ of diethyl ether is added to the residue, the ether layer is removed, and the residue is dissolved in 20 mℓ of water and chromatographed on a column of XAD-2. Elution is performed with 10 % aqueous ethanol. Fractions containing the desired compound are collected and concentrated under reduced pressure. The residue is lyophilized to give 0.2 g of the titled compound.

Example 21

7β-[2-(5-amino-1,2,4-thiadiazol-3-yl)-2(Z)-ethoxyiminoacetamido]-3-[[4-(1,2,4-oxadiazol-3-yl)pyridinium-1-yl]methyl]-3-cephem-4-carboxylate.

In 20 ml of a mixture of acetonitrile and water(1:1) are dissolved 2.0 g of 7β-[2-(5-amino-1,2,4-thiadiazol-3-yl)-2(Z)-ethoxyiminoacetamido]-3-(3-oxobutyryloxymethyl)-3-cephem-4-carboxylic acid, 2.0 g of 4-(1,2,4-oxadiazol-3-yl)-pyridine and 2.0 g of sodium iodide, and the mixture is stirred at 70°C for 2 hours. To the reaction mixture is added ethyl acetate and the organic layer is discarded after shaking. The aqueous layer is chromatographed on a column of silica gel. After washing the column with acetone, elution is effected with a mixture of acetone and water(7:3). Fractions containing the desired compound are collected and concentrated, and the residue is subjected to XAD-2 column chromatography. Elution is carried out with 10% aqueous ethanol and fractions containing the objective compound are collected and concentrated under reduced pressure. The residue is lyophilized to give the titled compound.

Elemental analysis, for $C_{21}H_{19}N_9O_6S_2 \cdot 3H_2O$:

Calcd.(%): C, 41.24; H, 4.12; N, 20.61.

Found (%): C, 41.09; H, 3.78; N, 20.73.

IR spectrum $\nu_{max}^{KBr} cm^{-1}$: 3300, 1760, 1620, 1520, 1380, 1040.

NMR spectrum ($d_6$-DMSO)δ: 1.25(3H,t,J=7Hz), 4.18(2H,q,J=7Hz), 4.98(1H,d,J=5Hz), 5.17 & 5.70(2H, ABq,J=13Hz), 5.68(1H,d.d,J=5Hz and 8Hz), 7.58(2H,d,J=6Hz), 8.16(2H,br.s), 8.74(2H,d,J=6Hz), 8.76(1H,s), 9.50(H,d,J=8Hz).

Example 22

7β-[2-(5-Amino-1,2,4-thiadiazol-3-yl)-2(Z)-ethoxyiminoacetamido]-3-[[4-(pyrazol-3-yl)pyridinium-1-yl]methyl]-3-cephem-4-carboxylate.

7β-[2-(5-Amino-1,2,4-thiadiazol-3-yl)-2(Z)-ethoxyiminoacetamido]-3-(3-oxobutyryloxymethyl)-3-cephem-4-carboxylic acid, 4-(pyrazol-3-yl)pyridine and sodium iodide are reacted in the same procedure as Example 21 to give the titled compound.

Elemental analysis, for $C_{22}H_{21}N_9O_5S_2 \cdot 7/2H_2O$:

Calcd.(%): C, 42.71; H, 4.56; N, 20.38

Found (%): C, 42.57; H, 4.13, N, 20.15.

IR spectrum $\nu_{max}^{KBr}cm^{-1}$: 3300, 1760, 1630, 1520, 1355, 1035

NMR spectrum ($d_6$-DMSO)δ: 1.20(3H,t,J=7Hz), 4.15(2H,q,J=7Hz), 5.10(1H,d,J=4.5Hz), 5.18 and 5.72(2H,ABq,J=14Hz), 5.70(1H,d.d,J=4.5Hz and 8Hz), 7.20-8.30(5H,m), 8.43(2H,d,J=6Hz), 9.36(2H,d,J=6Hz), 9.45(1H,d,J=8Hz).

Example 23

Five grams of 7β-[2-(2-aminothiazol-4-yl)-2(Z)-methoxyiminoacetamido]-3-[[4-(1,2,4-oxadiazol-3-yl)pyridinium-1-yl]methyl]-3-cephem-4-carboxylate is dissolved in 50 ml of physiological saline under mixing to produce a composition for injection.

What we claim is:

1. A cephem compound of the formula;

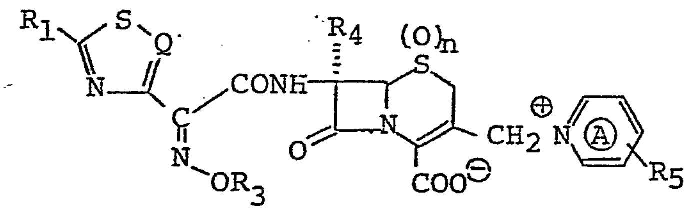

wherein $R_1$ is an optionally protected amino group; Q is a nitrogen atom or a C-$R_2$ group; $R_2$ is a hydrogen or halogen atom; $R_3$ is a hydrogen atom or an optionally substituted hydrocarbon residue; $R_4$ is a hydrogen atom or a methoxy group; $R_5$ is an optionally substituted nitrogen-containing aromatic heterocyclic group; Ⓐ denotes that further substituent or substituents may be present on the pyridine ring; n is 0 or 1 or its physiologically or pharmaceutically acceptable salt or ester.

2. A cephem compound according to claim 1 wherein the optionally substituted hydrocarbon residue is $C_{1-3}$ alkyl group which may be substituted with halogen atom, hydroxyl group, amino group, $C_{1-6}$ alkoxy group, carboxyl group, $C_{1-6}$ alkoxy-carbonyl group or cyano group.

3. A cephem compound according to claim 1 wherein the nitrogen-containing aromatic heterocyclic group is a five membered ring containing one to four nitrogen atoms and optionally one oxygen or sulfur atom.

4. A compound according to claim 1, namely 7β-[2-(2-aminothiazol-4-yl)-2(Z)-methoxyiminoacetamido]-3-[[4-(1,2,4-oxadiazol-3-yl)pyridinium-1-yl]methyl]-3-cephem-4-carboxylate.

5. A compound according to claim 1, namely 7β-[2-(2-aminothiazol-4-yl)-2(Z)-methoxyiminoacetamido]-3[[3-(1,3,4-oxadiazol-2-yl)pyridinium-1-yl]methyl]-3-cephem-4-carboxylate.

6. A compound according to claim 1, namely 7β-[2-(2-aminothiazol-4-yl)-2(Z)-methoxyiminoacetamido]-3-[[4-(pyrazol-3-yl)pyridinium-1-yl]methyl]-3-cephem-4-carboxylate.

7. A compound according to claim 1, namely 7β-[2-(2-aminothiazol-4-yl)-2(Z)-methoxyiminoacetamido]-3-[[3-(pyrazol-3-yl)pyridinium-1-yl]methyl]-3-cephem-4-carboxylate.

8. A compound according to claim 1, namely 7β-[2-(2-aminothiazol-4-yl)-2(Z)-methoxyiminoacetamido]-3-[[3-(imidazol-2-yl)pyridinium-1-yl]methyl]-3-cephem-4-carboxylate.

9. A compound according to claim 1, namely 7β-[2-(5-amino-1,2,4-thiadiazol-3-yl)-2(Z)-ethoxyiminoacetamido]-3[[4-(pyrazol-3-yl)pyridinium-1-yl]methyl]-3-cephem-4-carboxylate.

10. A process for producing a cephem compound of the formula;

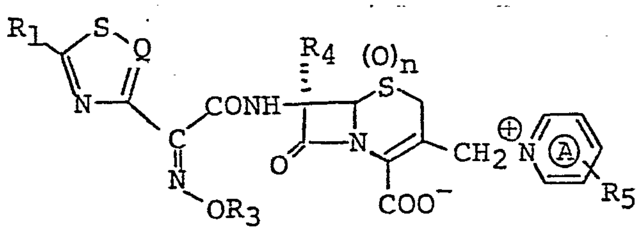

wherein $R_1$ is an optionally protected amino group; Q is a nitrogen atom or a C-$R_2$ group; $R_2$ is a hydrogen or halogen atom; $R_3$ is a hydrogen atom or an optionally substituted hydrocarbon residue; $R_4$ is a hydrogen atom or a methoxy group; $R_5$ is an optionally substituted nitrogen-containing aromatic heterocyclic group; Ⓐ denotes that further substituent or substituents may be present on the pyridine ring; n is 0 or 1 or its physiologically or pharmaceutically acceptable salt or ester, which comprises

(1) allowing a compound of the formula;

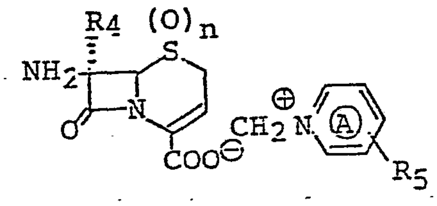

wherein the symbols are of the same meaning as defined above or its salt or ester to react with a compound of the formula;

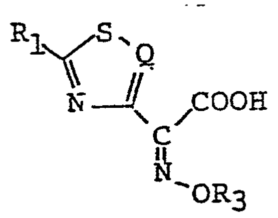

wherein the symbols are of the same meaning as defined above or its reactive derivative;

(2) allowing a compound of the formula;

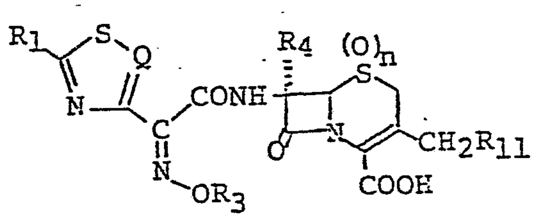

wherein $R_{11}$ is a hydrogen, acyloxy, carbamoyloxy or substituted carbamoyloxy group or a halogen atom; other symbols are as defined hereinbefore or its salt or ester to react with a compound of the formula 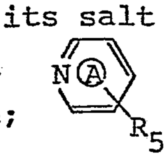 wherein the symbols are as defined hereinbefore;

(3) allowing a compound of the formula;

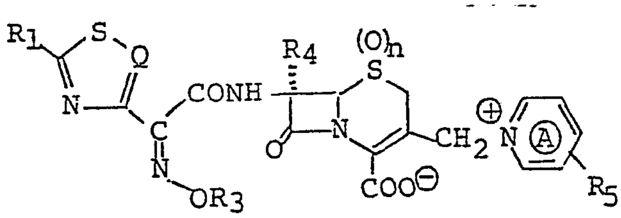

wherein the symbols are as defined hereinbefore or its salt or ester to react with a compound of the formula $R_{3'}OH$ wherein $R_{3'}$ is a hydrocarbon residue which may be substituted or its reactive derivative; or

(4) allowing a compound of the formula;

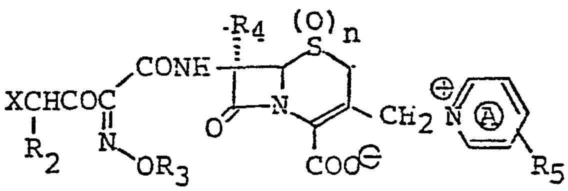

wherein X is a halogen atom or a group of the formula $R_6SO_2O$; $R_6$ is a $C_{1-4}$ alkyl group, pheny group or phenyl substituted with a $C_{1-4}$ alkyl group, $C_{1-6}$ alkoxy group, halogen atom or nitro group; other symbols are as defined hereinbefore or its salt or ester to react with a compound of the formula $R_1C(=S)NH_2$ wherein $R_1$ is as defined hereinbefore,

followed by removal of the protective group, if necessary.

11. A pharmaceutical composition produced by mixing at least one of the cephem compounds of the formula;

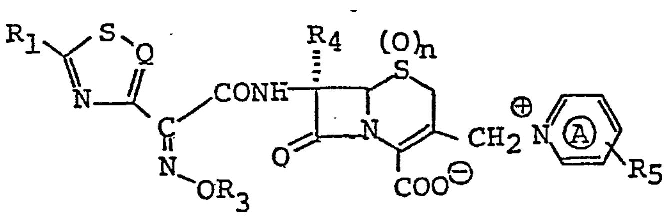

wherein $R_1$ is an optionally protected amino group; Q is a nitrogen atom or a $C\text{-}R_2$ group; $R_2$ is a hydrogen or halogen atom; $R_3$ is a hydrogen atom or an optionally substituted hydrocarbon residue; $R_4$ is a hydrogen atom or a methoxy group $R_5$ is an optionally substituted nitrogen-containing aromatic heterocyclic group; Ⓐ denotes that further subtituent or substituents may be present on the pyridine ring; n is 0 or 1 or their physiologically or pharmaceutically acceptable salts or esters and pharmaceutically acceptable carriers or excipients.